# EUROPEAN PATENT APPLICATION

(11) **EP 4 810 616 A1**
(43) Date of publication of application: **23.09.2026**
(21) Application number: 25901873.7
(22) Date of filing: 02.12.2025
(51) Int. Cl.: C12N 15/77, C12N 9/12, C07K 14/195, C12P 13/22, C12P 13/08, C12P 13/24

(54) **MICROORGANISM COMPRISING HETEROLOGOUS FRUCTOKINASE AND NON-PTS SUGAR UPTAKE FACTOR, AND METHOD FOR PRODUCING L-AMINO ACID USING SAME**

(30) Priority: 02.12.2024 KR 20240176886; 01.09.2025 KR 20250123532
(71) Applicant: CJ Cheiljedang Corporation, Seoul 04560 (KR)
(72) Inventor: YOON, Jeonghye, Seoul 04560 (KR); KIM, Ji-Won, Seoul 04560 (KR); JUNG, Moo Young, Seoul 04560 (KR); KIM, Kyungrim, Seoul 04560 (KR); KIM, Hyemi, Seoul 04560 (KR)
(74) Representative: Meissner Bolte Partnerschaft mbB
(86) International application number: PCT/KR2025/020410
(87) International publication number: WO 2026/121796

(57) **Abstract**

The present disclosure relates to a microorganism comprising a foreign fructokinase and non-PTS sugar transporter, and a method for producing an L-amino acid using the same.

## Description

### [Technical Field]

The present disclosure relates to a microorganism comprising a foreign fructokinase and a non-PTS sugar transporter, and a method for producing an L-amino acid using the same.

### [Background Art]

Processes for producing a desired substance (*e.g.*, an amino acid) in microorganisms are environmentally friendly and safe production methods and have been the subject of various studies. Among these, research on producing a desired substance in large quantities from microorganisms of the genus *Corynebacterium* has been continuously conducted. Microorganisms of the genus *Corynebacterium* are Gram-positive microorganisms widely used for the production of L-amino acids and other useful substances.

L-Amino acids are the fundamental building blocks of proteins and are used as important materials of pharmaceutical raw materials, food additives, animal feed, nutritional supplements, insecticides, and fungicides. In order to produce L-amino acids and other useful substances, various studies have been conducted on developing microorganisms and fermentation process technologies for high-efficiency production. For example, substance-specific approaches such as increasing the expression of genes encoding enzymes involved in the biosynthesis of L-tryptophan or eliminating genes unnecessary for the biosynthesis have mainly been employed (US 8,945,907 B2).

### [Disclosure]

### [Technical Problem]

The technical problem to be solved by the present disclosure is to provide a microorganism comprising a foreign fructokinase and a non-PTS sugar transporter, and a method for producing an L-amino acid using the same.

### [Technical Solution]

One example of the present disclosure is to provide a microorganism of the genus *Corynebacterium* that produces an L-amino acid, comprising: at least one selected from the group consisting of a fructokinase derived from a microorganism of the genus *Escherichia*, a polynucleotide encoding the same, a variant polypeptide of the fructokinase, or a polynucleotide encoding the variant polypeptide; and at least one selected from the group consisting of a non-PTS sugar transporter derived from a microorganism of the genus *Zymomonas*, or a polynucleotide encoding the same.

Another example of the present disclosure is to provide a composition for producing an L-amino acid, comprising the microorganism, a culture of the microorganism, a fermentation product of the microorganism, or a combination of two or more thereof.

Still another example of the present disclosure is to provide a method for producing an L-amino acid, comprising culturing the microorganism in a medium.

### [Advantageous Effects]

When culturing the microorganism comprising a foreign fructokinase and a non-PTS sugar transporter of the present disclosure, high-yield L-amino acid production can be achieved.

### [Detailed Description of Preferred Embodiments]

The present disclosure will be described in detail as follows. Meanwhile, each description and embodiment described herein may be applied to other descriptions and embodiments, respectively. That is, all combinations of the various elements disclosed herein fall within the scope of the present disclosure. Further, the scope of the present disclosure is not limited by the specific description described below.

Furthermore, those skilled in the art may recognize or identify numerous equivalents to the specific aspects of the present disclosure described herein by using routine experimentation. Moreover, these equivalents are intended to be comprised in the present disclosure.

Additionally, a number of papers and patent documents have been cited throughout the present specification. The content of the cited papers and patent documents is incorporated herein by reference in their entirety to describe the level of the technical field to which the present disclosure belongs and the contents of the present disclosure more clearly.

### Definitions

As used in the specification and appended claims of the present disclosure, the singular articles ("a", "an", and "the") comprise plural referents unless the context clearly indicates otherwise. Further, unless the context indicates otherwise, singular terms comprise their plural forms, and plural terms comprise their singular forms. Additionally, as used in the specification and appended claims of the present disclosure, the use of "or" may comprise the meaning of "and/or", unless indicated otherwise.

As used herein, the term "about" may precede a specific numerical value. As used herein, the term "about" encompasses not only the precise numerical value that is specified after the term but also a range that is approximately or nearly close to that value. Considering the context in which the number is presented, it can be determined whether the specific number mentioned is close to or nearly that number. In one example, the term "about" can refer to a range of -10% to +10% of the numerical value. As another example, the term "about" may refer to a range of -5% to +5% of the given numerical value. However, the term is not limited thereto.

As used herein, terms such as "first, second, third,...", "i), ii), iii),...", or "(a), (b), (c), (d),..." are used to distinguish similar elements. When these terms are used in relation to steps of a method, use, or analysis, these terms do not indicate that the steps are to be performed consecutively or in a particular order and the steps may be performed, for example, with no temporal interval between the steps, simultaneously, or sequentially, in reverse order, or in a random order with intervals of seconds, minutes, hours, days, or months.

As used herein, the term "consisting of" means that the ratio of the specific features, steps, components, or other element(s) following the term amounts to 100%. The features, steps, components, or other elements following the term "consisting of" may be essential or mandatory. For example, apart from the features, steps, components, or other elements following the term "consisting of", other arbitrary features, steps, components, or other elements, or non-essential features, steps, components, or other elements may be excluded.

As used herein, the term "consisting essentially of" may mean that, where the features, steps, components, or other elements claimed in the present disclosure are substantially unaffected by the presence of at least one unspecified feature, step, component, or another element, the at least one unspecified feature, step, component, or other element may be present.

As used herein, the term "comprising" refers to the presence of features, steps, components, or other elements following the term and does not exclude the presence of at least one additional feature, step, or component. As used herein, the specific features, steps, components, or other elements following the term "comprising" may be essential or mandatory; however, in some specific embodiments, other arbitrary or non-essential features, steps, components, or other elements may also be comprised.

### Protein, Polypeptide, Variant Protein, and Variant Polypeptide

As used herein, the term "protein" or "polypeptide" refers to a polymer or oligomer of consecutive amino acid residues. In the present disclosure, "polypeptide", "protein", and "peptide" may be used interchangeably.

As used herein, the term "mature polypeptide" or "mature protein" refers to a polypeptide or protein in a form without a signal sequence or propeptide sequence. A mature polypeptide or mature protein may be in a functional form of polypeptide or protein. Mature polypeptide or mature protein refers to the final form of polypeptide after translation and/or post-translational modification. For example, examples of the post-translational modification may include N-terminal processing, C-terminal truncation, glycosylation, phosphorylation, or leader sequence removal, but are not limited thereto.

In the present disclosure, amino acid sequences are described in the N-terminus to C-terminus direction unless otherwise indicated.

In the present disclosure, with respect to amino acid sequences, it is apparent that a polypeptide or protein "comprising" an amino acid sequence set forth in a specific sequence number, a polypeptide or protein "consisting of" an amino acid sequence set forth in a specific sequence number, or a polypeptide or protein "having" an amino acid sequence set forth in a specific sequence number may also comprise a polypeptide or protein in which certain amino acid(s) are deleted, modified, substituted, or added, as long as it has the same or corresponding activity as the polypeptide or protein consisting of the amino acid sequence of the sequence number. For example, the polypeptide or protein may also comprise, in the internal region or the region upstream or downstream (N-terminus or C-terminus) of the polypeptide or protein, a polypeptide or protein having additions or deletions of amino acid(s), naturally-occurring mutations, silent mutations, or conservative substitutions that do not alter the functions of the protein of the present disclosure.

Further, the scope of the polypeptide or protein of an amino acid sequence set forth in a specific sequence number may also comprise, for example, a polypeptide or protein conjugated to an N-terminal signal (or leader) sequence involved in the co-translational or post-translational translocation of the protein (polypeptide) or a polypeptide or protein conjugated with another sequence or linker so as to enable determination, purification, or synthesis of the polypeptide or protein.

As used herein, the term "conservative substitution" refers to the substitution of an amino acid with another amino acid having similar structural and/or chemical properties. Such an amino acid substitution may generally occur based on similarity in polarity, charge, solubility, hydrophobicity, hydrophilicity, and/or amphipathic nature of the residues. For example, positively charged (basic) amino acids comprise arginine, lysine, and histidine; negatively charged (acidic) amino acids comprise glutamate and aspartate; amino acids with nonpolar side chains (nonpolar amino acids) comprise glycine, alanine, valine, leucine, isoleucine, methionine, phenylalanine, tryptophan, and proline; and amino acids with polar or hydrophilic side chains (polar amino acids) comprise serine, threonine, cysteine, tyrosine, asparagine, and glutamine. In another example, amino acids with electrically charged side chains (electrically charged amino acids) comprise arginine, lysine, histidine, glutamate, and aspartate, and amino acids with electrically uncharged side chains (also referred to as uncharged amino acids or neutral amino acids) comprise glycine, alanine, valine, leucine, isoleucine, methionine, phenylalanine, tryptophan, proline, serine, threonine, cysteine, tyrosine, asparagine, and glutamine. In another example, phenylalanine, tryptophan, and tyrosine may be categorized as aromatic amino acids. In another example, valine, leucine, and isoleucine may be categorized as branched-chain amino acids (branched amino acids). In another example, 20 types of amino acids may be classified into five groups by size, starting from the group of amino acids with relatively small volumes: glycine, alanine, and serine; cysteine, proline, threonine, aspartate, and asparagine; valine, histidine, glutamate, and glutamine; isoleucine, leucine, methionine, lysine, and arginine; and phenylalanine, tryptophan, and tyrosine. However, the classification of amino acids is not limited thereto. Typically, conservative substitution may have little or no effect on the activity of a polypeptide or protein.

As used herein, the term "variant polypeptide", "variant protein", or "variant" refers to a polypeptide in which one or more amino acids are conservatively substituted and/or modified, resulting in an amino acid sequence different from the amino acid sequence of the variant prior to mutation, while retaining the functions or properties thereof. Such a variant can generally be identified by modifying one or more amino acids in the amino acid sequence of the polypeptide and evaluating the properties of the modified polypeptide. That is, the ability of a variant may be increased, unchanged, or reduced relative to that of the polypeptide before modification. Additionally, certain variants may comprise a variant in which one or more regions, such as an N-terminal leader sequence or a transmembrane domain, have been removed. Other variants may comprise a variant in which a portion has been removed from the N- terminus and/or C-terminus of the mature protein. The term "variant" may be used interchangeably with terms comprising modification, modified polypeptide, modified protein, mutant, mutein, and divergent, and is not limited as long as the terms are used to indicate mutation.

In addition, the variant may also comprise deletion or addition of amino acids that have minimal influence on the properties and secondary structure of a polypeptide. For example, a polypeptide may be conjugated with a signal (or leader) sequence at the N-terminus of a protein involved in the translocation of proteins co-translationally or post-translationally. Further, the polypeptide may also be conjugated with another sequence or linker to allow for identification, purification, or synthesis of the polypeptide.

As used herein, "position N" may comprise position N and an amino acid position corresponding to position N. Specifically, the term may comprise the amino acid position corresponding to an arbitrary amino acid residue in a mature polypeptide set forth in a specific amino acid sequence. The specific amino acid sequence may be the amino acid sequence of SEQ ID NO: 139.

As used herein, the term "corresponding to" refers to an amino acid residue at the position recited in a polypeptide, or an amino acid residue that is similar, identical, or homologous to the residue recited in a peptide. Identification of the amino acid at the corresponding position may be determining a specific amino acid in a sequence that references a particular sequence.

For example, based on the alignment of an arbitrary amino acid sequence with SEQ ID NO: 139, each amino acid residue in the amino acid sequence can be numbered with reference to the position numbers of the amino acid residues corresponding to the amino acid residues of SEQ ID NO: 139. For example, a sequence alignment algorithm as described herein can confirm the position of an amino acid or a position where modification such as substitution, insertion, or deletion occurs compared to a query sequence (also referred to as the "reference sequence").

For such an alignment, for example, the Needleman-Wunsch algorithm (Needleman and Wunsch, 1970, J. Mol. Biol. 48: 443-453), the Needleman program of the EMBOSS package (EMBOSS: The European Molecular Biology Open Software Suite, Rice et al., 2000, Trends Genet. 16: 276-277), and the like may be used, but are not limited thereto. Furthermore, a known sequence program, a pairwise sequence comparison algorithm, *etc.*, that is known in the art may be appropriately used.

### Gene, Polynucleotide

As used herein, the term "gene" refers, in a narrow sense, to a polynucleotide encoding a functional molecule, and, in a broad sense, to a polynucleotide comprising the polynucleotide encoding the functional molecule and a polynucleotide comprising region upstream or downstream of the polynucleotide. In one specific embodiment, the functional molecule may be an RNA or a protein, and a gene may have a sequence (intron) inserted between each coding region (exon).

As used herein, the term "polynucleotide" or "nucleic acid" refers to a polymer of nucleotides in which nucleotide monomers are connected in a long chain by covalent bonds, which is a DNA (*e.g.*, cDNA or genomic DNA) or RNA (*e.g.*, mRNA) strand having at least a certain length. In the present disclosure, "polynucleotide" and "nucleic acid" may be used interchangeably.

### Identity, Homology

As used herein, the term "identity" or "homology'" refers to the degree of similarity between two given amino acid sequences or base sequences and may be expressed as a percentage. In the present disclosure, "homology" and "identity" may often be used interchangeably.

The sequence homology or identity of conserved polynucleotides or polypeptides may be determined by a standard alignment algorithm, and default gap penalties established by the program used may be applied.

Whether any two polynucleotide or polypeptide sequences have homology or identity may be determined by, for example, a known computer algorithm such as the "FASTA" program (Pearson et al., (1988) [Proc. Natl. Acad. USA 85]: 2444) using default parameters. Alternatively, it may be determined by comparing the sequence information using the Needleman-Wunsch algorithm (Needleman and Wunsch, 1970, J. Mol. Biol. 48:443-453) as performed in the Needleman program of the EMBOSS package (EMBOSS: The European Molecular Biology Open Software Suite, Rice et al., 2000, Trends Genet. 16:276-277) (version 5.0.0 or later), or a GAP computer program such as the Smith-Waterman algorithm (Smith and Waterman, Adv. Appl. Math (1981) 2:482) (the GCG program package (Devereux, J. et al., Nucleic Acids Research 12:387 (1984)), BLASTP, BLASTN, and FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073) are included). For example, the homology or identity may be determined by using BLAST of the National Center for Biotechnology Information or ClustalW.

Further, whether any two polynucleotide sequences have homology or identity may be confirmed through Southern hybridization experiments under appropriate hybridization conditions, and the appropriate hybridization conditions can be determined by methods well known to those skilled in the art (such as J. Sambrook et al., Molecular Cloning, A Laboratory Manual; F.M. Ausubel et al., Current Protocols in Molecular Biology, John Wiley & Sons, Inc., New York), but are not limited thereto. For example, homologous or identical polynucleotide sequences can generally hybridize under stringent conditions along the whole sequence or at least about 50%, 60%, 70%, 80%, or 90% of the full length.

As used herein, the term "stringent conditions" refers to conditions that enable specific hybridization between polynucleotides. These conditions are specifically described in the literature (see Sambrook *et al.*, supra, 9.50-9.51, 11.7-11.8). For example, the stringent conditions may comprise conditions under which polynucleotides having high homology or identity, *i.e.*, polynucleotides having at least 60%, at least 70%, at least 75%, at least 80%, at least 85%, at least 90%, at least 95%, at least 96%, at least 97%, at least 98%, or at least 99% homology or identity hybridize with each other, while polynucleotides having homology or identity lower than the above do not hybridize with each other; or may comprise ordinary washing conditions of Southern hybridization, *i.e.*, washing once, specifically twice or three times, at a salt concentration and temperature corresponding to 60°C, 1×SSC, 0.1% SDS, specifically 60°C, 0.1×SSC, 0.1% SDS, and more specifically 68°C, 0.1×SSC, 0.1% SDS.

The hybridization may occur between nucleotides having complementary base sequences, but the hybridized polynucleotides may comprise some base mismatches depending on the stringency of the hybridization. The term "complementary" is used to describe a relationship between nucleotide bases that can hybridize with each other. For example, regarding DNA, adenosine is complementary to thymine, and cytosine is complementary to guanine. Therefore, the polynucleotide of the present disclosure may comprise an isolated nucleic acid fragment complementary to the entire sequence as well as a base sequence substantially similar thereto.

Specifically, polynucleotides having homology or identity to the polynucleotide of the present disclosure may be detected through hybridization at a Tₘ value of 55°C Further, the Tₘ value may be 60°C, 63°C, or 65°C, but is not limited thereto, and may be appropriately adjusted by those skilled in the art.

The appropriate stringency for hybridizing the polynucleotides depends on the length and degree of complementarity of the polynucleotides, and these variables are well known in the art (*e.g.*, Sambrook *et al.*, supra).

### Vector, Transformation

As used herein, the term "vector" refers to a DNA construct for delivering a desired polynucleotide into a suitable host or host cell.

In one example, the vector may comprise a base sequence of a polynucleotide encoding a desired polypeptide that is operably linked to a suitable expression regulatory region (or expression regulatory sequence) so as to enable the expression of the desired polypeptide in a suitable host. The expression regulatory sequence may comprise a promoter capable of initiating transcription, any operator sequence for regulating the transcription, a sequence encoding a suitable mRNA ribosome-binding site, and a sequence for regulating termination of transcription and translation. The vector, after being transformed into a suitable host cell (microorganism), may replicate or function independently of the host genome, or may be integrated into the genome itself to replicate or function.

Additionally, in one example, the vector of the present disclosure may comprise a sequence for inserting a desired polynucleotide into a chromosome. The insertion of a polynucleotide into a chromosome using a vector may be performed by any method known in the art, for example, homologous recombination, but is not limited thereto.

The vector used in the present disclosure is not particularly limited, and any vector known in the art may be used. Examples of commonly used vectors comprise plasmids, cosmids, viruses, and bacteriophages, either in their natural state or in a recombinant state. For example, as a phage vector or cosmid vector, pWE15, M13, MBL3, MBL4, IXII, ASHII, APII, t10, t11, Charon4A, Charon21A, *etc.*, may be used; and as a plasmid vector, those based on pDZ, pDC, pBR, pUC, pBluescriptII, pGEM, pTZ, pCL, pET, *etc.*, may be used. In one example, pDZ, pDC, pDC24, pACYC177, pACYC184, pCL, pECCG117, pUC19, pBR322, pMW118, pCC1BAC vectors, *etc.*, may be used.

The vector may further comprise a selection marker to confirm transformation into a host cell, or further, insertion into a chromosome of the host cell. The selection marker is used to select cells transformed with the vector or to confirm the insertion of a desired polynucleotide into a chromosome; markers that confer selectable phenotypes, such as drug resistance, nutrient requirements, resistance to cytotoxic agents, or expression of surface polypeptides, may be used. In an environment treated with a selective agent, only the cells that express the selection marker survive or exhibit a different phenotype, allowing for the selection of transformed cells.

As used herein, the term "transformation" refers to the introduction of a desired polynucleotide or a vector comprising the same into a host cell (microorganism), thereby altering the genetic traits of the host cell (microorganism). The transformed polynucleotide may be inserted into the chromosome of a host cell or located outside the chromosome. Further, the polynucleotide may comprise DNA and/or RNA. The polynucleotide may be introduced in a suitable form depending on the purpose of introduction. For example, a polynucleotide for expressing a desired polypeptide may be introduced into a host cell (microorganism) in the form of an expression cassette, which is a gene construct comprising all elements required for self-expression. The expression cassette may typically comprise a promoter operably linked to the coding sequence of a desired polynucleotide, a transcription termination signal, a ribosome binding site, and a translation termination signal. The expression cassette may be in the form of an expression vector capable of self-replication. Further, the polynucleotide may be introduced into a host cell (microorganism) as-is and may be operably linked to a sequence required for expression in the host cell (microorganism), but is not limited thereto.

As used herein, the term "operably linked" refers to a configuration in which a regulatory sequence is positioned at an appropriate position to regulate the expression of a coding sequence. Thus, the term "operably linked" comprises attaching or linking a regulatory region of a functional domain having a known or desired activity such as a promoter, a stop codon, a signal sequence, or an enhancer region, with a target (gene or polypeptide) so as to regulate the expression, secretion, or function of the target in accordance with the known or desired activity. For example, it may mean that a polynucleotide sequence encoding a polypeptide is functionally linked to a promoter sequence that initiates and mediates transcription of the polynucleotide.

As used herein, the term "expression" comprises any step involved in the production of a polypeptide, for example, transcription, post-transcriptional modification, translation, post-translational modification, and secretion, but is not limited thereto.

As used herein, the term "expression vector" refers to a linear or circular nucleic acid molecule comprising a desired polynucleotide sequence and an operably linked regulatory sequence for expression thereof. For example, it may comprise a base sequence of a polynucleotide encoding a desired polypeptide that is operably linked to a suitable expression regulatory region (or expression regulatory sequence) such that the desired polypeptide can be expressed in a suitable host.

As used herein, the term "regulatory sequence" refers to a polynucleotide sequence necessary for regulating the expression of a desired polynucleotide sequence. Each regulatory sequence may be a regulatory sequence native (derived from the same origin) or foreign (derived from a different gene) to the coding sequence, a variant sequence thereof, or another artificial sequence. Examples of the regulatory sequence may comprise a leader sequence, a polyadenylation sequence, a propeptide sequence, a promoter, a signal peptide sequence, an operator sequence, a sequence encoding a ribosome-binding domain, and a sequence regulating the termination of transcription and translation. The minimum unit of the regulatory sequence may comprise a promoter and a sequence for terminating transcription and translation.

As used herein, the term "genetic recombination" refers to a natural or artificial process in which an element constituting a gene, such as DNA or RNA, is rearranged into an order different from the original sequence during disassembly and reassembly processes.

As used herein, the term "recombinant gene" refers to a gene having a new genomic configuration that results from genetic recombination, such as chemical synthesis or genetic engineering techniques. In the present disclosure, the terms "recombinant gene", "recombinant DNA", and "recombinant polynucleotide" may be used interchangeably. In one example, the recombinant gene may comprise an artificial combination of nucleic acid fragments such as regulatory sequences that are not naturally found together.

As used herein, the term "recombinant protein" refers to a protein produced as a result of genetic recombination.

### Microorganism

As used herein, the term "microorganism (or strain)" includes both wild-type microorganisms and prokaryotic or eukaryotic microorganisms that have undergone genetic modification naturally or artificially. It may be a microorganism in which a specific mechanism is weakened or increased due to the insertion of a foreign gene, or an increase in or inactivation of activity of an endogenous gene, and may be a microorganism comprising a genetic modification for the production of a polypeptide, protein, or product of interest. In the present disclosure, the terms "microorganism", "strain", "host", and "host cell" may be used interchangeably.

As used herein, the term "recombinant microorganism" refers to a microorganism that has been genetically modified to exhibit a different genotype and/or phenotype compared to a naturally occurring microorganism (*e.g.*, when genetic modification affects how the nucleic acid sequence is encoded in the microorganism), and may comprise all progeny or potential progeny of the microorganism. In the present disclosure, the terms "recombinant microorganism", "genetically modified microorganism", "recombinant host cell", "recombinant cell", and "recombinant strain" may be used interchangeably. The recombinant microorganism may, for example, express a gene that is not found in its natural (non-recombinant) form, not express a gene that is expressed in its natural form, or express a natural gene in a manner different from that in which it is expressed in its natural form.

For example, the microorganism of the present disclosure may comprise a microorganism (*e.g.*, recombinant microorganism) into which at least one selected from the group consisting of a fructokinase derived from a microorganism of the genus *Escherichia*, a polynucleotide encoding the same, a variant polypeptide of the fructokinase, and a polynucleotide encoding the variant polypeptide; and at least one selected from the group consisting of a non-PTS sugar transporter derived from a microorganism of the genus *Zymomonas*, and a polynucleotide encoding the same are inserted, but is not limited thereto.

As used herein, the term "microorganism having L-amino acid-producing ability" refers to a microorganism that is capable of producing an L-amino acid within an organism, and may comprise any microorganism that inherently lacks L-amino acid-producing ability but is imparted with L-amino acid-producing ability or any microorganism inherently having L-amino acid-producing ability. The ability to produce an L-amino acid may be imparted or enhanced by strain improvement.

As used herein, the term "non-modified microorganism (strain)" does not exclude a microorganism (strain) comprising mutations that may occur naturally, and may refer to a wild-type microorganism (strain) or a natural microorganism (strain) per se, or a microorganism (strain) before a trait is altered by genetic variation due to natural or artificial factors. In the present disclosure, the term "non-modified microorganism (strain)" may be used interchangeably with "microorganism (strain) before modification", "non-mutated microorganism (strain)", "parent microorganism", "parent strain", "wild-type microorganism (strain)", "reference microorganism (strain)", or "standard microorganism (strain)". In the present disclosure, the non-modified microorganism may mean a microorganism (strain) that does not comprise at least one selected from the group consisting of a fructokinase derived from a microorganism of the genus *Escherichia*, a polynucleotide encoding the same, a variant polypeptide of the fructokinase, and a polynucleotide encoding the variant polypeptide; and/or at least one selected from the group consisting of a non-PTS sugar transporter derived from a microorganism of the genus *Zymomonas*, and a polynucleotide encoding the same, or a microorganism (strain) before the insertion of the protein or polynucleotide, but is not limited thereto. In one example, the non-modified microorganism in the present disclosure may be a microorganism of the genus *Corynebacterium* that does not comprise a polypeptide consisting of the amino acid sequence of SEQ ID NO: 139, SEQ ID NO: 147, SEQ ID NO: 148, and/or SEQ ID NO: 140 or a polynucleotide encoding the same, but is not limited thereto.

### Increased Activity of Protein (Polypeptide)

As used herein, an "increase" in protein (polypeptide) activity means that the activity of a protein (polypeptide) is increased compared to its intrinsic activity in a host cell (microorganism). The increase may be used interchangeably with terms such as "activation", "up-regulation", "overexpression", or "enhancement". The host cell (microorganism) may be a prokaryotic or eukaryotic microorganism.

The increase in protein (polypeptide) activity may comprise both the case in which a host cell (microorganism) exhibits a protein (polypeptide) activity that it did not inherently possess, and the case in which a host cell exhibits an improved protein (polypeptide) activity compared to its intrinsic activity or its activity before modification.

For example, the case in which it "exhibits a protein (polypeptide) activity that it did not inherently possess" or the case in which it exhibits an improved protein (polypeptide) activity may result from "introduction of a protein (polypeptide)", but is not limited thereto.

As used herein, the term "introduction" of a protein (polypeptide) refers to a case in which a gene that a microorganism did not originally possess is expressed within the microorganism so that the activity of a specific protein is exhibited, or to a case in which the polypeptide activity is enhanced, increased, or improved compared to its intrinsic activity or its activity prior to modification. For example, it may result from the introduction of a gene encoding the protein (polypeptide) into a host cell (microorganism). For example, a polynucleotide encoding a specific protein (polypeptide) may be introduced into a chromosome of a host cell (microorganism), or a vector comprising the polynucleotide encoding a specific protein (polypeptide) may be introduced into a host cell (microorganism), thereby exhibiting or enhancing the activity thereof.

The "intrinsic activity" refers to the activity of a specific protein (polypeptide) originally possessed by a host cell (microorganism) before transformation or a non-modified host cell (microorganism), when the trait is altered by genetic modification due to natural or artificial factors. This term may be used interchangeably with "activity before modification".

An increase in activity of a protein (polypeptide) compared to its intrinsic activity means that the activity and/or concentration (expression level) of the protein (polypeptide) in a host cell (microorganism) is enhanced compared to the activity and/or concentration (expression level) of the protein (polypeptide) originally possessed by the host cell (microorganism) before modification or a non-modified host cell (microorganism).

In one example, the increase may refer to the emergence of activity of the corresponding protein (polypeptide) that was absent, or the increase in activity or concentration of the protein, relative to the activity or concentration in the host cell (microorganism) before transformation or in a non-modified host cell (microorganism), typically by at least about 1%, at least about 10%, at least about 25%, at least about 50%, at least about 75%, at least about 100%, at least about 150%, at least about 200%, at least about 300%, at least about 400%, or at least about 500%, up to at least about 1000% or at least about 2000% or more, but is not limited thereto.

The increase in activity of a protein (polypeptide) may be achieved by introducing a foreign protein (polypeptide) or by increasing the activity of an endogenous protein (polypeptide). Whether the activity of a protein (polypeptide) has increased may be confirmed from the increase in the activity level of the protein (polypeptide), its expression level, or the amount of product resulting from the activity of the corresponding protein (polypeptide).

The increase in the activity of a protein (polypeptide) can be achieved by various methods well known in the art, and is not limited as long as the activity of the desired protein (polypeptide) can be increased compared to that of the host cell (microorganism) before modification. Specifically, it may be achieved using genetic engineering and/or protein engineering well known to those skilled in the art, which are routine molecular biology techniques, but is not limited thereto (*e.g.*, Sitnicka et al. Functional Analysis of Genes. Advances in Cell Biology. 2010, Vol. 2. 1-16, Sambrook et al. Molecular Cloning 2012, *etc.*).

Specifically, the increase in the activity of a protein (polypeptide) of the present disclosure may result from:
1) increase in the intracellular copy number of a polynucleotide encoding the protein (polypeptide);
2) modification of an expression regulatory region of a gene encoding the protein (polypeptide) on the chromosome (*e.g.*, introduction of a modification on the expression regulatory region, replacement with a sequence exhibiting stronger activity, or insertion of a sequence exhibiting stronger activity);
3) modification of a base sequence encoding a start codon or the 5'-UTR of a gene transcript encoding the protein (polypeptide);
4) modification of the amino acid sequence of the protein (polypeptide) to increase the activity of the protein (polypeptide);
5) modification of a polynucleotide sequence encoding the protein (polypeptide) to increase the activity of the protein (polypeptide) (*e.g.*, modification of a polynucleotide sequence of a gene encoding the protein (polypeptide), such that the gene encodes a protein (polypeptide) modified to have increased activity);
6) introduction of a foreign protein (polypeptide) exhibiting the activity of the protein (polypeptide), or a foreign polynucleotide encoding the same;
7) codon optimization of a polynucleotide encoding the protein (polypeptide);
8) selection and modification or chemical modification of exposed regions of the protein (polypeptide) through analysis of the tertiary structure thereof;
9) regulation of the cellular localization of the protein (polypeptide); or
10) a combination of two or more selected from 1) to 9) above, but is not particularly limited thereto.

For example,
The 1) increase in the intracellular copy number of a polynucleotide encoding the protein (polypeptide) may be achieved by introducing a vector comprising the polynucleotide encoding the protein (polypeptide) operably linked to a suitable regulatory sequence into a host cell (microorganism). Alternatively, it may be achieved by introducing one copy, or two or more copies, of a polynucleotide encoding the protein (polypeptide) operably linked to a suitable regulatory sequence into a chromosome of a host cell (microorganism). The introduction into a chromosome may be performed by introducing a vector capable of inserting the polynucleotide into a chromosome of a host cell (microorganism) into the host cell (microorganism), but is not limited thereto. The vector is as described above. With respect to the polynucleotide sequence encoding the protein, the regulatory sequence may be a native sequence (of the same origin) or a foreign sequence (derived from a different gene), a variant thereof, or another artificial sequence, and may induce the expression of the polynucleotide in a host cell (microorganism).
The 2) replacement of the expression regulatory region (or expression regulatory sequence) of a gene encoding the protein (polypeptide) on the chromosome with a sequence having strong activity may be achieved, for example, by introducing a modification into the sequence through deletion, insertion, substitution, or a combination thereof to further increase the activity of the expression regulatory region, or by replacing the sequence with a sequence having stronger activity. The expression regulatory region may comprise, but is not particularly limited to, a promoter, an operator sequence, a sequence encoding a ribosome-binding site, a sequence regulating the termination of transcription and translation, and the like. In one example, it may specifically comprise replacing the original promoter with a strong promoter, but is not limited thereto.

Examples of known strong promoters comprise cj1 to cj7 promoters (US 7,662,943 B2), lac promoter, trp promoter, trc promoter, tac promoter, lambda phage PR promoter, PL promoter, tet promoter, gapA promoter, SPL7 promoter, SPL13 (sm3) promoter (US 10,584,338 B2), O2 promoter (US 10,273,491 B2), tkt promoter, yccA promoter, and the like, but the strong promoter is not limited thereto.

The 3) modification of a base sequence encoding a start codon or the 5'-UTR of a gene transcript encoding the protein (polypeptide) may be achieved, for example, by modifying the base sequence such that it encodes a different initiation codon with a higher expression rate of the protein (polypeptide) compared to the endogenous initiation codon, or by modifying the base sequence such that it encodes a ribosome binding site (RBS) sequence with a higher expression rate of the protein (polypeptide) compared to the endogenous RBS sequence, but is not limited thereto.

The 4) and 5) modification of the amino acid sequence or polynucleotide sequence of the protein (polypeptide) may be achieved by introducing a modification on the sequence of the amino acid sequence of the protein (polypeptide) or the polynucleotide sequence encoding the protein (polypeptide) through deletion, insertion, substitution, or a combination thereof to increase the activity of the protein (polypeptide), or by replacing the sequence with an amino acid sequence or polynucleotide sequence modified to increase the activity, but is not limited thereto. The replacement may specifically be performed by inserting the polynucleotide into a chromosome by homologous recombination, but is not limited thereto.

The 6) introduction of a foreign polynucleotide exhibiting the activity of the protein (polypeptide) may be achieved by introducing into a host cell (microorganism) a foreign polynucleotide encoding a protein (polypeptide) that exhibits the same/similar activity to that of the protein (polypeptide). The foreign polynucleotide is not limited by its origin or sequence, as long as it exhibits the same/similar activity to that of the protein (polypeptide). The introduction may be performed by a transformation method known in the art appropriately selected by those skilled in the art, and the expression of the introduced polynucleotide in the host cell enables the production of the protein (polypeptide), thereby increasing its activity.

The 7) codon optimization of a polynucleotide encoding the protein (polypeptide) may be achieved by codon-optimizing an endogenous polynucleotide to increase the transcription or translation within a host cell (microorganism), or by optimizing the codons of a foreign polynucleotide such that optimized transcription and translation thereof may be achieved within the host cell.

The 8) selection and modification or chemical modification of exposed regions of the protein (polypeptide) through analysis of the tertiary structure thereof may be achieved, for example, by comparing the sequence information of the protein (polypeptide) to be analyzed with a database storing the sequence information of known proteins to identify template protein candidates according to the degree of sequence similarity and confirming the structure based on the information, thereby selecting the exposed site to be modified or chemically modified and transforming or modifying the same.

The 9) regulation of the cellular localization of the protein (polypeptide) may be achieved by targeting the protein (polypeptide) to a particular intracellular organelle or a particular intracellular space. For example, it may be achieved by targeting the protein (polypeptide) to the periplasm or cytoplasm through the addition or removal of a leader sequence that functions in the targeting of proteins (polypeptides), but is not limited thereto.

Such an increase in protein (polypeptide) activity may refer to an increase in the activity or concentration of the corresponding protein (polypeptide) relative to the activity or concentration of the protein (polypeptide) expressed in a wild-type strain or a host cell (microorganism) before modification, or an increase in the amount of product produced by the protein (polypeptide), but is not limited thereto.

### Reduced Activity of Protein (Polypeptide)

As used herein, "reduction" in protein (polypeptide) activity is a concept that encompasses any activity of a protein (polypeptide) that is decreased compared to its intrinsic activity in a host cell (microorganism) or inactivated. That is, the reduction in the activity of a protein (polypeptide) may comprise protein (polypeptide) activity reduced compared to the intrinsic activity or activity before modification of the protein (polypeptide), where the protein (polypeptide) is not completely inactivated; or activity of a protein (polypeptide) that is completely inactivated.

For example, the above reduction may comprise the case where the protein (polypeptide) activity is lower or absent compared to the protein (polypeptide) possessed by the host cell (microorganism) before transformation or a non-modified host cell due to mutations in the polynucleotide encoding the protein (polypeptide); the case where the overall level of protein (polypeptide) expression within the cell is lower compared to the host cell (microorganism) before transformation or a non-modified host cell due to inhibition of the expression of the polynucleotide or protein (polypeptide); the case where no expression of the polynucleotide or protein (polypeptide) occurs; and the case where the protein (polypeptide) activity is low or absent despite normal protein (polypeptide) expression.

The "intrinsic activity" refers to the activity of a specific protein (polypeptide) originally possessed by a host cell (microorganism) before transformation or a non-modified host cell (microorganism), when the trait is altered by genetic modification due to natural or artificial factors. This term may be used interchangeably with "activity before modification".

The host cell (microorganism) may be a prokaryotic or eukaryotic microorganism.

A reduction in activity of a protein (polypeptide) compared to its intrinsic activity means that the activity and/or concentration (expression level) of the protein (polypeptide) in a host cell (microorganism) is reduced compared to the activity and/or concentration (expression level) of the protein (polypeptide) originally possessed by the host cell (microorganism) before modification or a non-modified host cell (microorganism).

Whether the activity of a protein (polypeptide) has been reduced may be confirmed from the increase in the activity level of the protein (polypeptide), its expression level, or the amount of product resulting from the activity of the corresponding protein (polypeptide).

In one example, the above reduction may be a reduction in the activity or concentration of the corresponding protein (polypeptide) to about less than 100%, about 90% or less, about 80% or less, about 70% or less, about 60% or less, about 50% or less, about 40% or less, about 30% or less, about 20% or less, about 10% or less, about 5% or less, or 0% based on the activity or concentration in the host cell (microorganism) before transformation or a non-modified host cell (microorganism), but is not limited thereto.

The reduction in the activity of a protein (polypeptide) can be achieved by various methods well known in the art, and is not limited as long as the activity of the desired protein (polypeptide) can be reduced compared to that of the host cell (microorganism) before modification. Specifically, it may be achieved using genetic engineering and/or protein engineering well known to those skilled in the art, which are routine molecular biology techniques, but is not limited thereto (*e.g.*, Nakashima N et al., Bacterial cellular engineering by genome editing and gene silencing. Int J Mol Sci. 2014;15(2):2773-2793; Sambrook et al. Molecular Cloning 2012; *etc.*).

Specifically, the reduction in the activity of a protein (polypeptide) of the present disclosure may result from:
1) deletion of all or part of a gene encoding the protein (polypeptide);
2) modification of a gene expression regulatory region on a chromosome encoding the protein (polypeptide) (*e.g.*, introduction of a modification on the expression regulatory region, replacement with a sequence exhibiting expression inhibitory activity, or insertion of a sequence exhibiting expression inhibitory activity);
3) modification of the amino acid sequence of the protein (polypeptide) so as to reduce the activity of the protein (polypeptide) (*e.g.*, deletion/substitution/addition of one or more amino acids in the amino acid sequence);
4) modification of a polynucleotide sequence encoding the protein (polypeptide) to reduce the activity of the protein (polypeptide) (*e.g.*, modification of a polynucleotide sequence of a gene encoding the protein (polypeptide), such that the gene encodes a protein (polypeptide) modified to have reduced activity);
5) modification of a base sequence encoding a start codon or the 5'-UTR of a gene encoding the protein (polypeptide);
6) introducing an antisense oligonucleotide (*e.g.*, antisense RNA) that binds complementarily to the gene transcript encoding the protein (polypeptide);
7) addition of a sequence complementary to the Shine-Dalgarno (SD) sequence upstream of the SD sequence of a gene encoding the protein (polypeptide) to form a secondary structure that prevents ribosome attachment;
8) reverse transcription engineering (RTE), which adds a promoter to be reversely transcribed on the 3' terminus of the open reading frame (ORF) of a polynucleotide sequence encoding the protein (polypeptide); or
9) regulation of the cellular localization of the protein (polypeptide); or
10) a combination of two or more selected from 1) to 9) above, but is not particularly limited thereto.

For example,
The 1) deletion of all or part of a gene encoding a protein (polypeptide) may be achieved by using a method using homologous recombination via a chromosomal insertion vector in a microorganism, or by using electromagnetic waves, such as ultraviolet rays, X rays, or gamma rays, or chemicals, but is not limited thereto.
The 2) replacement of the expression regulatory region encoding the protein (polypeptide) on the chromosome with a sequence having expression inhibitory activity may be achieved, for example, by introducing a modification into the sequence on the expression regulatory region through deletion, insertion, substitution, or a combination thereof to further reduce the expression inducing activity of the expression regulatory region, or by replacing the sequence with a sequence having expression inhibitory activity. The expression regulatory region may include, but is not particularly limited to, a promoter, an operator sequence, a sequence encoding a ribosome binding site, and a sequence regulating the termination of transcription and translation.
The 3) and 4) modification of the amino acid sequence or polynucleotide sequence of the protein (polypeptide) may be achieved by introducing a modification on the sequence of the amino acid sequence of the protein (polypeptide) or the polynucleotide sequence encoding the protein (polypeptide) through deletion, insertion, substitution, or a combination thereof to reduce the activity of the protein (polypeptide), or by replacing the sequence with an amino acid sequence or polynucleotide sequence modified to have reduced activity, but is not limited thereto. The modification of the sequence may specifically be performed by inserting the polynucleotide of the modified sequence into a chromosome by homologous recombination, but is not limited thereto. In one specific embodiment, by introducing a mutation into a polynucleotide sequence encoding the protein (polypeptide) to form a stop codon, the protein (polypeptide) may be inactivated, but is not limited thereto.
The 5) modification of a base sequence encoding a start codon or the 5'-UTR of a gene transcript encoding the protein (polypeptide) may be achieved, for example, by substituting the start codon with a different start codon with a lower expression rate of the protein (polypeptide) compared to the endogenous initiation codon, or by modifying the base sequence such that it encodes a ribosome binding site (RBS) sequence with a lower expression rate of the protein (polypeptide) compared to the endogenous RBS sequence, but is not limited thereto.
The 6) introduction of an antisense oligonucleotide (*e.g.*, antisense RNA) that binds complementarily to the gene transcript encoding the protein (polypeptide) may be performed in reference to the literature [Weintraub, H. et al., Antisense-RNA as a molecular tool for genetic analysis, Reviews - Trends in Genetics, Vol. 1(1) 1986], but is not limited thereto.
The 7) addition of a sequence complementary to the Shine-Dalgarno (SD) sequence upstream of the SD sequence of a gene encoding the protein (polypeptide) to form a secondary structure that prevents ribosome attachment may be achieved by inhibiting mRNA translation or reducing the speed thereof, but is not limited thereto.
The 8) reverse transcription engineering (RTE), which adds a promoter to be reversely transcribed on the 3' terminus of the open reading frame (ORF) of a polynucleotide sequence encoding the protein (polypeptide), may be achieved by generating an antisense nucleotide complementary to the gene transcript encoding the polypeptide to inhibit the translation of the protein (polypeptide), thereby reducing the activity.
The 9) regulation of the cellular localization of the protein (polypeptide) may be achieved by targeting the protein (polypeptide) to a particular intracellular organelle or a particular intracellular space. For example, it may be achieved by targeting the protein (polypeptide) to the periplasm or cytoplasm through the addition or removal of a leader sequence that functions in the targeting of proteins (polypeptides), but is not limited thereto.

Such reduction of the protein (polypeptide) activity may refer to a reduction in the activity or concentration of the corresponding protein (polypeptide) relative to the activity or concentration of the protein (polypeptide) expressed in a wild-type strain or a host cell (microorganism) before modification, but is not limited thereto.

The modification of a part or the entirety of a polynucleotide in the host cell (microorganism) of the present disclosure may be induced by: (a) a method using homologous recombination using a vector for chromosomal insertion or genome editing using an engineered nuclease (*e.g.*, CRISPR-Cas9), and/or (b) treatment with light, such as ultraviolet light and radiation, and/or chemicals, but is not limited thereto.

### Culturing

As used herein, the term "culturing" refers to growing a microorganism under suitably controlled environmental conditions. The culturing process may be performed in a suitable medium known in the art under suitable culturing conditions known in the art. Such a culturing process may be easily adjusted and used by those skilled in the art according to the selected microorganism. Specifically, the culturing may be batch culturing, continuous culturing, and/or fed-batch culturing, but is not limited thereto.

As used herein, the term "medium" refers to a mixed substance containing nutrients required for culturing a microorganism as a main component, and the medium supplies nutrients, growth factors, *etc.*, including water, which are indispensable for survival and development. Specifically, any medium and culture conditions may be used for culturing the microorganism of the present disclosure without particular limitation, as long as the medium is used for the common culture of microorganisms. For example, the microorganism of the present disclosure may be cultured in a common medium containing appropriate carbon sources, nitrogen sources, phosphorus sources, inorganic compounds, amino acids, and/or vitamins, *etc.*, while controlling the temperature, pH, *etc.* under aerobic conditions. For example, a culture medium for a microorganism of the genus *Corynebacterium* can be found in the document ["Manual of Methods for General Bacteriology" by the American Society for Bacteriology (Washington D.C., USA, 1981)].

In the present disclosure, the carbon sources comprise carbohydrates such as glucose, saccharose, lactose, fructose, sucrose, and maltose; sugar alcohols such as mannitol and sorbitol; organic acids such as pyruvic acid, lactic acid, and citric acid; amino acids such as glutamate, methionine, and lysine, and the like. Additionally, natural organic nutrients such as starch hydrolysates, molasses, blackstrap molasses, rice bran, cassava, sugarcane bagasse, and corn steep liquor may be used. Specifically, carbohydrates such as glucose and sterilized pretreated molasses (*i.e.*, molasses converted into reducing sugars) may be used, and other various carbon sources in appropriate amounts may be used without limitation. These carbon sources may be used alone or in a combination of two or more thereof, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate, or organic nitrogen sources such as amino acids such as glutamic acid, methionine, and glutamine, peptone, NZ-amine, meat extracts, yeast extract, malt extract, corn steep liquor, casein hydrolysate, fish or decomposition products thereof, and skim soybean cake or decomposition products thereof may be used. These nitrogen sources may be used alone or in a combination of two or more thereof, but are not limited thereto.

As the phosphorus sources, monobasic potassium phosphate, dibasic potassium phosphate, or the corresponding sodium-containing salts may be used. As for inorganic compounds, sodium chloride, calcium chloride, iron chloride, magnesium sulfate, iron sulfate, manganese sulfate, calcium carbonate, *etc.*, may be used, and in addition, amino acids, vitamins, and/or suitable precursors may be comprised. These components or precursors may be added to the medium in a batchwise or continuous manner. However, the medium is not limited thereto.

During the culturing of the microorganism of the present disclosure, compounds such as ammonium hydroxide, potassium hydroxide, ammonia, phosphoric acid, and sulfuric acid may be added to the medium in a suitable manner to adjust the pH of the medium. During the culturing, an antifoaming agent such as fatty acid polyglycol ester may be used to suppress foaming. To maintain an aerobic state of the medium, oxygen or oxygen-containing gas may be injected into the medium. To maintain an anaerobic or microaerobic state of the medium, no gas may be injected, or nitrogen, hydrogen, or carbon dioxide gas may be injected. However, the culturing conditions are not limited thereto.

In the culturing of the present disclosure, the culturing temperature may be maintained at 20°C to 45°C, specifically 25°C to 40°C, and the culturing may be performed for about 10 to 160 hours, but the culturing conditions are not limited thereto.

As used herein, the term "culture" refers to a culture solution, a concentrated culture solution, a dried product of the culture solution, a culture filtrate, a concentrated culture filtrate, or a dried product of the culture filtrate obtained by culturing a specific microorganism in a culture medium. The culture solution refers to a solution comprising a specific microorganism, whereas the culture filtrate refers to a solution that substantially does not contain the specific microorganism (wherein "substantially" means that the specific microorganism separated by filtration or the like is excluded, but does not mean that the microorganism is completely absent from the filtrate). The culture is not limited in its form, and, in one example, may be in the form of a liquid, an emulsion, or a solid.

As used herein, the term "fermentation" refers to a process in which a microorganism decomposes organic matter using enzymes possessed by the microorganism, excluding putrefactive reactions. Fermentation reactions and putrefactive reactions proceed using similar processes. However, upon decomposition, fermentation produces useful substances, whereas putrefaction produces bad odors or harmful substances.

In the present disclosure, the method for obtaining a fermentation product from the microorganism is not particularly limited and can be obtained according to a method conventionally used in the relevant or similar technical field.

As used herein, the term "fermentation product" comprises not only the fermented substance itself, but also all types of substances comprising a fermentation product produced from the microorganism, comprising a substance comprising the fermented microorganism, a culture produced from the fermented microorganism, a fermentation product of the culture, a concentrated fermentation product, a dried product of the fermentation product, a filtrate of the fermentation product, a filtrate of the concentrated fermentation product, a dried product of the filtrate of the fermentation product, an extract of the fermentation product, or a dilution of the fermentation product.

### Detailed Description of the Present Disclosure

Hereinafter, the embodiments of the present disclosure will be described in more detail as follows.

One aspect of the present disclosure provides a microorganism of the genus *Corynebacterium* that produces an L-amino acid, comprising: at least one selected from the group consisting of a fructokinase derived from a microorganism of the genus *Escherichia*, a polynucleotide encoding the same, a variant polypeptide of the fructokinase, and a polynucleotide encoding the variant polypeptide; and at least one selected from the group consisting of a non-PTS sugar transporter derived from a microorganism of the genus *Zymomonas*, and a polynucleotide encoding the same.

As used herein, the term "fructokinase" is a protein having the activity of phosphorylating fructose.

It is known that in a microorganism that expresses fructokinase, sucrose is broken down into 6-phosphorylated glucose and fructose by invertase, then fructose is phosphorylated by fructokinase, and 6-phosphorylated glucose and phosphorylated fructose are used in glycolysis.

In one example, the fructokinase of the present disclosure may be a protein having fructokinase activity encoded by the *cscK* gene, but is not particularly limited to any type, as long as it has activity corresponding to the fructokinase. In one example, the fructokinase of the present disclosure may be derived from *Escherichia coli*. In one example, the fructokinase of the present disclosure may be encoded by the *cscK* gene of *Escherichia coli*.

The cscK derived from a microorganism of the genus *Escherichia coli* is a fructokinase gene belonging to the csc regulon group and is known to be involved in sucrose metabolism along with cscB (proton symport-type sucrose permease) and cscA (sucrose hydrolase) (J. Bacteriol., 184: 5307-5316, 2002).

The fructokinase encoded by the *cscK* gene is known in the art, and the amino acid and polynucleotide sequences of the fructokinase can be obtained from publicly available databases, examples of which include GenBank of NCBI, but are not limited thereto.

In one example, the fructokinase protein derived from a microorganism of the genus *Escherichia* may comprise the amino acid sequence of SEQ ID NO: 139 or an amino acid sequence having 60% or more homology or identity thereto, but is not limited thereto as long as it has fructokinase activity. Additionally, it is apparent that even if a protein comprises an amino acid sequence of SEQ ID NO: 139 in which part of the sequence is deleted, modified, substituted, or added, the protein may fall within the scope of the fructokinase of the present disclosure as long as the protein exhibits efficacy corresponding to that of the fructokinase. A protein having, comprising, consisting of, or essentially consisting of an amino acid sequence having at least 60%, 70%, 80%, 85%, 90%, 95%, 96%, 97%, 98%, or 99% or more homology or identity to the amino acid sequence of SEQ ID NO: 139 that exhibits equivalent efficacy to that of the fructokinase may be comprised in the fructokinase.

Additionally, the sequence of a polynucleotide encoding fructokinase derived from a microorganism of the genus *Escherichia* having the amino acid sequence of SEQ ID NO: 139 or an amino acid sequence having at least 60% homology or identity thereto can be obtained, for example, based on codon information known in the art. In one example, the fructokinase may be encoded by a polynucleotide having, comprising, consisting of, or essentially consisting of the sequence of SEQ ID NO: 11 or a base sequence having at least 60%, at least 70%, at least 75%, at least 80%, at least 85%, at least 90%, at least 95%, at least 96%, at least 97%, at least 98%, or at least 99% homology or identity to the sequence of SEQ ID NO: 11, but is not limited thereto. The base sequence of SEQ ID NO: 11 can be obtained from a known database, such as GenBank of NCBI, but is not limited thereto.

In the present disclosure, the term "polynucleotide (gene) comprising the base sequence of SEQ ID NO: 11" can be used interchangeably with the term "polynucleotide (gene) having the base sequence of SEQ ID NO: 11", "polynucleotide (gene) consisting of the base sequence of SEQ ID NO: 11", or "*cscK*".

The variant polypeptide or fructokinase variant polypeptide of the present disclosure refers to a variant polypeptide in which the amino acid at position 79 or 181 in the amino acid sequence of SEQ ID NO: 139 is substituted with another amino acid.

The "fructokinase variant polypeptide" may be referred to as "fructokinase variant", "variant CscK", "CscK variant", "variant polypeptide", *etc.* The fructokinase polypeptide to which the variant of the present disclosure is introduced may be used interchangeably with the term "CscK" and is not particularly limited, but may be encoded by the *cscK* gene and may be CscK derived from *Escherichia coli*, but is not limited thereto.

The fructokinase variant polypeptide of the present disclosure may mean a variant polypeptide in which the amino acid at a position corresponding to position 79 or 181 in the amino acid sequence of SEQ ID NO: 139 is substituted with another amino acid.

The "another amino acid" or "other amino acid" is not limited as long as the amino acids differ from the amino acid before the substitution. Meanwhile, in the present disclosure, when it is expressed that "a specific amino acid has been substituted", it may be substituted with an amino acid different from the amino acid before substitution, even if it is not separately indicated that it has been substituted with a different amino acid.

In one example, the variant polypeptide may have an amino acid substitution at the position corresponding to position 79 in the amino acid sequence of SEQ ID NO: 139 with an amino acid other than tryptophan, which is the amino acid before substitution, or an amino acid substitution at the position corresponding to position 181 in the amino acid sequence of SEQ ID NO: 139 with an amino acid other than alanine, which is the amino acid before substitution, or a combination thereof. The variant may be one in which the amino acid at the position corresponding to position 79 in the amino acid sequence of SEQ ID NO: 139 is substituted with an amino acid selected from the group consisting of asparagine, valine, glycine, leucine, arginine, alanine, methionine, threonine, glutamine, proline, isoleucine, serine, phenylalanine, histidine, cysteine, tyrosine, lysine, aspartate, and glutamic acid, but is not limited thereto.

The variant may be one in which the amino acid at the position corresponding to position 181 in the amino acid sequence of SEQ ID NO: 139 is substituted with an amino acid selected from the group consisting of asparagine, valine, glycine, leucine, arginine, tryptophan, methionine, threonine, glutamine, proline, isoleucine, serine, phenylalanine, histidine, cysteine, tyrosine, lysine, aspartate, and glutamic acid, but is not limited thereto.

In one example, the variant polypeptide may have an amino acid substitution at the position corresponding to position 79 in the amino acid sequence of SEQ ID NO: 139 with cysteine, or an amino acid substitution at the position corresponding to position 181 in the amino acid sequence of SEQ ID NO: 139 with valine, or a combination thereof.

In one example, the variant polypeptide provided in the present disclosure may comprise an amino acid sequence having at least 60%, 65%, 70%, 75%, 80%, 85%, 90%, 95%, 96%, 97%, 98%, 99%, 99.5%, or 99.8% or more homology or identity with SEQ ID NO: 139.

In one example, the variant polypeptide provided in the present disclosure may have the amino acid corresponding to position 79 in the amino acid sequence set forth in SEQ ID NO: 139 fixed as cysteine, and may comprise an amino acid sequence having at least 60%, 65%, 70%, 75%, 80%, 85%, 90%, 95%, 96%, 97%, 98%, 99%, 99.5%, or 99.8% or more homology or identity with SEQ ID NO: 139. In addition, the variant polypeptide provided in the present disclosure may have the amino acid corresponding to position 181 in the amino acid sequence set forth in SEQ ID NO: 139 fixed as valine, and may comprise an amino acid sequence having at least 60%, 65%, 70%, 75%, 80%, 85%, 90%, 95%, 96%, 97%, 98%, 99%, 99.5%, or 99.8% or more homology or identity with SEQ ID NO: 139. In addition, the variant polypeptide provided in the present disclosure may have the amino acid corresponding to position 79 in the amino acid sequence set forth in SEQ ID NO: 139 fixed as cysteine and the amino acid corresponding to position 79 in the amino acid sequence set forth in SEQ ID NO: 139 fixed as valine, and may comprise an amino acid sequence having at least 60%, 65%, 70%, 75%, 80%, 85%, 90%, 95%, 96%, 97%, 98%, 99%, 99.5%, or 99.8% or more homology or identity with SEQ ID NO: 139. In addition, as long as it is an amino acid sequence having such homology or identity and exhibiting efficacy corresponding to the variant polypeptide of the present disclosure, it may be a variant polypeptide having an amino acid sequence with deletion, modification, substitution, conservative substitution, or addition in a part thereof, but is not limited thereto.

Meanwhile, those skilled in the art can identify the amino acid corresponding to position 79 or 181 of the amino acid sequence of SEQ ID NO: 139 of the present disclosure in any amino acid sequence through sequence alignment known in the art, and even if not particularly described in the present disclosure, if "an amino acid at a specific position in a specific SEQ ID NO" is disclosed, it may comprise up to "an amino acid at a position corresponding" thereto in any amino acid sequence, but is not limited thereto.

In one example, the variant polypeptide may consist of the amino acid sequence of SEQ ID NO: 147 or SEQ ID NO: 148.

Specifically, the variant polypeptide of the present disclosure may have, comprise, consist of, or essentially consist of an amino acid sequence having at least 60%, 70%, 80%, 85%, 90%, 95%, 96%, 97%, 98%, or 99% homology or identity to SEQ ID NO: 147 or SEQ ID NO: 148.

Specifically, the variant polypeptide of the present disclosure may have, comprise, consist of, or essentially consist of the amino acid sequence of SEQ ID NO: 147 or an amino acid sequence having at least 60%, 70%, 80%, 85%, 90%, 95%, 96%, 97%, 98%, or 99% homology or identity to SEQ ID NO: 147.

Specifically, the variant polypeptide of the present disclosure may have, comprise, consist of, or essentially consist of the amino acid sequence of SEQ ID NO: 148 or an amino acid sequence having at least 60%, 70%, 80%, 85%, 90%, 95%, 96%, 97%, 98%, or 99% homology or identity to SEQ ID NO: 148.

For example, the amino acid sequence may have sequence additions or deletions that do not alter the function of the variant of the present disclosure, naturally occurring mutations, silent mutations, or conservative substitutions.

The polynucleotide encoding the fructokinase variant polypeptide of the present disclosure may comprise a polynucleotide sequence without limitation, as long as it is a polynucleotide sequence encoding a variant polypeptide having fructokinase activity.

For example, the polynucleotide encoding the fructokinase variant polypeptide of the present disclosure may be a polynucleotide sequence encoding the amino acid sequence of the fructokinase variant polypeptide of the present disclosure, but is not limited thereto.

For example, it may comprise a nucleic acid sequence encoding the amino acid sequence set forth in SEQ ID NO: 147 or SEQ ID NO: 148. As an example of the present disclosure, the polynucleotide of the present disclosure may have or comprise SEQ ID NO: 149 or SEQ ID NO: 150. In addition, the polynucleotide of the present disclosure may consist of or essentially consist of SEQ ID NO: 149 or SEQ ID NO: 150.

For example, the polynucleotide of the present disclosure may comprise, in a base sequence having homology or identity of 60%, 70%, 75%, 80%, 85%, 90%, 95%, 96%, 97%, 98%, 99%, 99.5%, 99.7%, or 99.8% or more with respect to the sequence of SEQ ID NO: 11, substitution of a codon encoding tryptophan, which is the amino acid corresponding to positions 235 to 237 of SEQ ID NO: 11, with a codon encoding an amino acid other than tryptophan, for example, cysteine; or substitution of a codon encoding alanine, which is the amino acid corresponding to positions 541 to 543 of SEQ ID NO: 11, with a codon encoding an amino acid other than alanine, for example, valine; or both, but is not limited thereto.

In addition, it is apparent that a variant having a polynucleotide sequence in which deletion, modification, substitution, conservative substitution, or addition in a part thereof is comprised falls within the scope of the present disclosure, provided that the polynucleotide sequence has such homology or identity and encodes the amino acid sequence of the fructokinase variant polypeptide of the present disclosure.

In the present disclosure, the non-PTS sugar transporter is a protein having the activity of uptaking external sugars into a cell without consuming phosphoenolpyruvate (PEP).

In one example, the non-PTS sugar transporter of the present disclosure may have glucose and/or fructose uptake ability.

Specifically, the non-PTS sugar transporter may have an activity of transporting glucose and/or fructose into the cell, but is not limited thereto.

In a microorganism, a sugar uptake protein can be classified mainly into ATP-binding cassette (ABC) transporters, the Major Facilitator Superfamily (MFS), and the phosphoenolpyruvate(PEP):carbohydrate phosphotransferase system (PTS). PTS is also known as the phosphoenolpyruvate (PEP) dependent phosphotransferase transport system and is a sugar transport system in bacteria that is distinct from non-PTS systems.

The ATP-binding cassette transporter is characterized by requiring ATP for sugar molecules to enter the cell. In the case of MFS, it is characterized by comprising H+-linked symporters, Na+-linked symporters-antiporters, or uniporters. For example, an H⁺-symporter requires an extracellular proton for a sugar molecule to enter the cell. These ABC transporters and MFS are referenced as examples of the non-PTS sugar transporter. However, the non-PTS sugar transporter is not limited to these examples.

In one example, the non-PTS sugar transporter of the present disclosure may be a protein having sugar uptake protein activity encoded by the *glf* gene, and is not particularly limited in type as long as it has sugar uptake activity without consuming PEP and can be comprised in the non-PTS sugar transporter. In one example, the non-PTS sugar transporter of the present disclosure may be derived from *Zymomonas mobilis*. In one example, the non-PTS sugar transporter of the present disclosure may be encoded by the *glf* gene of *Zymomonas mobilis*, but is not limited thereto.

The non-PTS sugar transporter encoded by the *glf* gene is known in the art, and the amino acid and polynucleotide sequences of the non-PTS sugar transporter can be obtained from publicly available databases, examples of which include GenBank of NCBI, but are not limited thereto.

In one example, the non-PTS sugar transporter derived from a microorganism of the genus *Zymomonas* may comprise the amino acid sequence of SEQ ID NO: 140 or an amino acid sequence having 60% or more homology or identity thereto, but is not limited thereto as long as it has sugar uptake activity without consuming PEP. Additionally, it is apparent that even if a protein comprises an amino acid sequence of SEQ ID NO: 140 in which part of the sequence is deleted, modified, substituted, or added, the protein may fall within the scope of the non-PTS sugar transporter of the present disclosure as long as the protein exhibits efficacy corresponding to that of the non-PTS sugar transporter. A protein having, comprising, consisting of, or essentially consisting of an amino acid sequence having at least 60%, 70%, 80%, 85%, 90%, 95%, 96%, 97%, 98%, or 99% or more homology or identity to the amino acid sequence of SEQ ID NO: 140 that exhibits equivalent efficacy to that of the non-PTS sugar transporter may be comprised in the non-PTS sugar transporter.

Additionally, the sequence of a polynucleotide encoding the non-PTS sugar transporter derived from a microorganism of the genus *Zymomonas* having the amino acid sequence of SEQ ID NO: 140 or an amino acid sequence having at least 60% homology or identity thereto can be obtained, for example, based on codon information known in the art. In one example, the non-PTS sugar transporter may be encoded by a polynucleotide having, comprising, consisting of, or essentially consisting of the sequence of SEQ ID NO: 35 or a base sequence having at least 60%, at least 70%, at least 75%, at least 80%, at least 85%, at least 90%, at least 95%, at least 96%, at least 97%, at least 98%, or at least 99% homology or identity to the sequence of SEQ ID NO: 35, but is not limited thereto. The base sequence of SEQ ID NO: 35 can be obtained from a known database, such as GenBank of NCBI, but is not limited thereto.

In the present disclosure, the term "polynucleotide (gene) comprising the base sequence of SEQ ID NO: 35" can be used interchangeably with the term "polynucleotide (gene) having the base sequence of SEQ ID NO: 35", "polynucleotide (gene) consisting of the base sequence of SEQ ID NO: 35", or "*glf*".

The polynucleotide of the present disclosure may undergo various modifications in the coding region within the scope that do not change the amino acid sequence of the protein of the present disclosure, taking into consideration codon degeneracy or the codons preferred in an organism in which the protein of the present disclosure is to be expressed. Accordingly, it is apparent that a polynucleotide that can be translated, by codon degeneracy, into a polypeptide consisting of an amino acid sequence of the fructokinase, fructokinase variant polypeptide, and/or non-PTS sugar transporter of the present disclosure or a polypeptide having at least 60% homology or identity thereto may also be comprised in the polynucleotide of the present disclosure. For example, the polynucleotide of the present disclosure may be SEQ ID NO: 11, SEQ ID NO: 35, SEQ ID NO: 149, SEQ ID NO: 150, or a degenerate sequence thereof.

In another example, the polynucleotide of the present disclosure may have or comprise a base sequence having at least 60%, at least 70%, at least 75%, at least 80%, at least 85%, at least 90%, at least 95%, at least 96%, at least 97%, at least 98%, or at least 99% homology or identity to the sequence of SEQ ID NO: 11, SEQ ID NO: 35, SEQ ID NO: 149, or SEQ ID NO: 150, or may consist of or essentially consist of a base sequence having at least 60%, at least 70%, at least 75%, at least 80%, at least 85%, at least 90%, at least 95%, at least 96%, at least 97%, at least 98%, or at least 99% homology or identity to the sequence of SEQ ID NO: 11, SEQ ID NO: 35, SEQ ID NO: 149, or SEQ ID NO: 150, but is not limited thereto.

In addition, the polynucleotide of the present disclosure may comprise, without limitation, a probe that can be prepared from a known gene sequence, for example, a sequence encoding the fructokinase, the fructokinase variant polypeptide, and/or the non-PTS sugar transporter of the present disclosure prepared by hybridizing under stringent conditions with a sequence complementary to all or part of the polynucleotide sequence of the present disclosure.

The microorganism of the genus *Corynebacterium* of the present disclosure has L-amino acid-producing ability.

The microorganism of the present disclosure may comprise any microorganism that is capable of producing a desired L-amino acid by comprising: at least one selected from the group consisting of a fructokinase derived from a microorganism of the genus *Escherichia*, a polynucleotide encoding the same, a variant polypeptide of the fructokinase, and a polynucleotide encoding the variant polypeptide; and at least one selected from the group consisting of a non-PTS sugar transporter derived from a microorganism of the genus *Zymomonas*, and a polynucleotide encoding the same.

For example, the microorganism of the present disclosure may be a microorganism or a recombinant microorganism characterized by having increased L-amino acid-producing ability by comprising at least one selected from the group consisting of a fructokinase derived from a microorganism of the genus *Escherichia*, a polynucleotide encoding the same, a variant polypeptide of the fructokinase, and a polynucleotide encoding the variant polypeptide; and at least one selected from the group consisting of a non-PTS sugar transporter derived from a microorganism of the genus *Zymomonas*, and a polynucleotide encoding the same, and genetically modified by introducing the protein or polynucleotide thereinto, but is not limited thereto.

Specifically, the recombinant strain with increased L-amino acid-producing ability may be a microorganism with increased L-amino acid-producing ability compared to a non-modified microorganism that does not comprise a fructokinase derived from a natural wild-type microorganism or a microorganism of the genus *Escherichia*, or a polynucleotide encoding the same; and a non-PTS sugar transporter derived from a microorganism of the genus *Zymomonas*, or a polynucleotide encoding the same, but is not limited thereto.

In one example, the microorganism having L-amino acid-producing ability is a microorganism capable of producing an L-amino acid within a living organism, and may comprise any microorganism that inherently has L-amino acid-producing ability or a microorganism into which L-amino acid-producing ability is imparted by the activity of the fructokinase derived from the genus *Escherichia* of the present disclosure or a variant polypeptide thereof; and a non-PTS sugar transporter derived from the genus *Zymomonas* in a parent strain that does not have L-amino acid-producing ability. The ability to produce an L-amino acid may be imparted or enhanced by strain improvement.

The microorganism of the present disclosure may comprise any microorganism into which at least one selected from the group consisting of a fructokinase derived from a microorganism of the genus *Escherichia*, a polynucleotide encoding the same, a variant polypeptide of the fructokinase, and a polynucleotide encoding the variant polypeptide; and at least one selected from the group consisting of a non-PTS sugar transporter derived from a microorganism of the genus *Zymomonas*, and a polynucleotide encoding the same are inserted by various known methods.

In one example, the recombinant microorganism having L-amino acid-producing ability of the present disclosure may comprise any microorganism capable of producing an L-amino acid by introducing a foreign gene encoding the fructokinase derived from a microorganism of the genus *Escherichia* of the present disclosure or a variant polypeptide thereof, and a non-PTS sugar transporter derived from a microorganism of the genus *Zymomonas* through transformation using a vector.

For example, the microorganism producing an L-amino acid may be a microorganism into which a polynucleotide encoding a fructokinase comprising the amino acid sequence of SEQ ID NO: 139 or a fructokinase comprising an amino acid sequence having at least 60%, 70%, 75%, 80%, 85%, 90%, 95%, 96%, 97%, 98%, 99%, 99.5%, 99.7%, or 99.9% or more homology or identity to the amino acid sequence of SEQ ID NO: 139 is introduced.

For example, the microorganism that produces an L-amino acid may be a microorganism into which a polynucleotide sequence encoding a fructokinase variant polypeptide comprising a sequence in which the amino acid corresponding to position 79 or 181 of SEQ ID NO: 139 is substituted with a different amino acid; or a fructokinase variant polypeptide comprising substitution of the amino acid corresponding to position 79 of SEQ ID NO: 139 with cysteine, substitution of the amino acid corresponding to position 181 of SEQ ID NO: 139 with valine, or a combination of the above; or a fructokinase variant polypeptide comprising an amino acid sequence having at least 60%, 70%, 75%, 80%, 85%, 90%, 95%, 96%, 97%, 98%, 99%, 99.5%, 99.7%, or 99.9% or more homology or identity to the amino acid sequence of SEQ ID NO: 147 or 148 is introduced.

For example, the microorganism producing an L-amino acid may be a microorganism into which a polynucleotide capable of encoding a protein comprising an amino acid sequence having at least 60% homology with the amino acid sequence of SEQ ID NO: 139; or a polynucleotide comprising the base sequence of SEQ ID NO: 11 or a base sequence having at least 60%, at least 70%, at least 75%, at least 80%, at least 85%, at least 90%, at least 95%, at least 96%, at least 97%, at least 98%, or at least 99% homology or identity to the base sequence of SEQ ID NO: 11 is introduced.

For example, the microorganism producing an L-amino acid may be a microorganism into which a polynucleotide capable of encoding a protein comprising an amino acid sequence having at least 60% homology with the amino acid sequence of SEQ ID NO: 147 or 148; or a polynucleotide comprising the base sequence of SEQ ID NO: 149 or 150, or a base sequence having at least 60%, at least 70%, at least 75%, at least 80%, at least 85%, at least 90%, at least 95%, at least 96%, at least 97%, at least 98%, or at least 99% homology or identity to the base sequence of SEQ ID NO: 149 or 150 is introduced.

For example, the microorganism producing an L-amino acid may be a microorganism into which a polynucleotide encoding a non-PTS sugar transporter comprising the amino acid sequence of SEQ ID NO: 140 or a non-PTS sugar transporter comprising an amino acid sequence having at least 60%, 70%, 75%, 80%, 85%, 90%, 95%, 96%, 97%, 98%, 99%, 99.5%, 99.7%, or 99.9% or more homology or identity to the amino acid sequence of SEQ ID NO: 140 is introduced.

For example, the microorganism producing an L-amino acid may be a microorganism into which a polynucleotide capable of encoding a protein comprising an amino acid sequence having at least 60% homology with the amino acid sequence of SEQ ID NO: 140; or a polynucleotide comprising the base sequence of SEQ ID NO: 35 or a base sequence having at least 60%, at least 70%, at least 75%, at least 80%, at least 85%, at least 90%, at least 95%, at least 96%, at least 97%, at least 98%, or at least 99% homology or identity to the base sequence of SEQ ID NO: 35 is introduced.

In one example, the microorganism with increased L-amino acid-producing ability of the present disclosure may be a microorganism with increased L-amino acid-producing ability compared to a non-modified microorganism, but is not limited thereto. In one example, the non-modified microorganism, which is the reference strain for comparing whether the L-amino acid-producing ability has increased, may be the ATCC13869, CA04-8357, KCCM11016P, ATCC 13032, CM05-9841, CJH1, or KCCM 12120P strain, but is not limited thereto.

In one example, the recombinant microorganism having increased L-amino acid-producing ability may exhibit an increase of at least about 1%, specifically at least about 1%, at least about 2.5%, at least about 5%, at least about 6%, at least about 7%, at least about 8%, at least about 9%, at least about 10%, at least about 15%, at least about 16%, at least about 17%, at least about 18%, at least about 19%, at least about 20%, or at least about 21% (the upper limit is not particularly limited and may be, for example, at most about 200%, at most about 150%, at most about 100%, at most about 50%, at most about 45%, at most about 40%, at most about 35%, at most about 30%, or at most about 25%) compared to the L-amino acid-producing ability of a parent strain before modification or a non-modified microorganism, but is not limited thereto. In another example, the microorganism having increased L-amino acid-producing ability may have an increase of at least about 1.1-fold, at least about 1.15-fold, at least about 1.16-fold, at least about 1.17-fold, at least about 1.18-fold, at least about 1.19-fold, at least about 1.2-fold, or at least about 1.21-fold (the upper limit is not particularly limited, for example, at most about 10-fold, at most about 5-fold, at most about 3-fold, at most about 2-fold, at most about 1.5-fold, at most about 1.4-fold, at most about 1.3-fold, or at most about 1.25-fold) compared to that of a parent microorganism (parent strain) before modification or a non-modified microorganism, but is not limited thereto.

The microorganism of the present disclosure may be a microorganism of the genus *Corynebacterium*.

In one example of the present disclosure, the microorganism of the present disclosure may be *Corynebacterium glutamicum*, *Corynebacterium crudilactis*, *Corynebacterium deserti*, *Corynebacterium efficiens*, *Corynebacterium callunae*, *Corynebacterium stationis*, *Corynebacterium singulare*, *Corynebacterium halotolerans*, *Corynebacterium striatum*, *Corynebacterium pollutisoli*, *Corynebacterium imitans*, *Corynebacterium testudinoris*, or *Corynebacterium flavescens*. Specifically, the microorganism of the present disclosure may be *Corynebacterium glutamicum*, but is not limited thereto.

Meanwhile, the microorganism of the genus *Corynebacterium* having L-amino acid-producing ability of the present disclosure comprises any of a natural wild-type microorganism, a microorganism of the genus *Corynebacterium* with improved L-amino acid-producing ability by enhancing or weakening the activity of a gene related to the branched chain amino acid production mechanism, or a microorganism of the genus *Corynebacterium* with L-amino acid-producing ability by introducing or enhancing the activity of a foreign gene.

In one example, the microorganism of the present disclosure may have reduced activity of the PEP-dependent phosphotransferase system (PTS). For example, part or all of the activity of the proteins constituting the phosphotransferase system that uptakes fructose may be reduced. In one example, the microorganism of the present disclosure may have reduced expression of a pts gene. In one example, the expression of the *ptsF* gene may be reduced, or the expression may be reduced due to a deletion of the *ptsF* gene, but is not limited thereto.

In one example, the microorganism of the present disclosure may comprise a modification that increases L-tryptophan-producing ability. The microorganism modified to increase L-tryptophan-producing ability may have enhanced expression of the gene encoding anthranilate synthase, the tryptophan operon, and transketolase with released feedback inhibition. The microorganism modified to increase L-tryptophan-producing ability may comprise a TrpE comprising an S38R substitution at a position corresponding to position 38 from the N-terminus of *Corynebacterium* TrpE or comprising a P21S substitution at a position corresponding to position 21 from the N-terminus of *Escherichia* TrpE. The disclosure of KR 10-2035844 B1 is incorporated herein by reference with respect to a microorganism having an improved tryptophan biosynthetic pathway.

In one example, the microorganism of the present disclosure may comprise a modification that increases the L-histidine-producing ability. The microorganism modified to increase L-histidine-producing ability may comprise HisG (ATP phosphoribosyltransferase) with released feedback inhibition. The HisG may comprise, for example, substitutions corresponding to G233H and T235Q of HisG from *Corynebacterium glutamicum*, the contents of which are described in ACS Synth. Biol., 2014, 3(1), pp. 21-29, which is incorporated herein by reference. The microorganism modified to increase L-histidine-producing ability may have enhanced expression of hisE, hisG, hisN, hisD, hisA, hisH, and hisB. The expression enhancement can be achieved, for example, through replacement of the start codon, replacement of the promoter, or an increase in the gene copy number.

In one example, the microorganism of the present disclosure may comprise a modification that increases the L-threonine-producing ability. The microorganism modified to increase L-threonine-producing ability may comprise aspartate kinase (LysC) and homoserine dehydrogenase (Hom) with released feedback inhibition. The LysC may comprise, for example, a substitution corresponding to L377K of the LysC from *Corynebacterium glutamicum*. The Hom may comprise, for example, a substitution corresponding to R398Q of the Hom from *Corynebacterium glutamicum*. With respect to a microorganism having increased threonine-producing ability, the disclosure of US 11,236,374 B2 is incorporated herein by reference.

Still another aspect of the present disclosure provides a method for producing an L-amino acid, comprising culturing, in a medium, a microorganism of the genus *Corynebacterium* comprising: at least one selected from the group consisting of a fructokinase derived from a microorganism of the genus *Escherichia*, a polynucleotide encoding the same, a variant polypeptide of the fructokinase, and a polynucleotide encoding the variant polypeptide; and at least one selected from the group consisting of a non-PTS sugar transporter derived from a microorganism of the genus *Zymomonas*, and a polynucleotide encoding the same.

In the method for producing an L-amino acid of the present disclosure, culturing of the microorganism may be performed using any culturing conditions and culturing methods known in the art. Such a culturing process may be readily adjusted and used by those skilled in the art according to the selected strain.

The L-amino acid produced by the culturing of the present disclosure may be secreted into the medium or remain in the cells.

The L-amino acid of the present disclosure comprises any L-amino acid that can be produced by a microorganism to which the production ability has been imparted, or that can be inherently produced through metabolic pathways from various carbon sources. Specifically, the L-amino acid may be a basic amino acid such as L-lysine, L-arginine, or L-histidine; a non-polar amino acid such as L-valine, L-leucine, L-glycine, L-isoleucine, L-alanine, L-proline, or L-methionine; a polar amino acid such as L-serine, L-threonine, L-cysteine, L-asparagine, or L-glutamine; an aromatic amino acid such as L-phenylalanine, L-tyrosine, or L-tryptophan; or an acidic amino acid such as L-glutamic acid or L-aspartic acid. In one example, in the present disclosure, the L-amino acid may be selected from L-tryptophan, L-lysine, L-histidine, and L-threonine.

In one example, the method for producing an L-amino acid of the present disclosure may further comprise preparing the microorganism of the present disclosure, preparing a medium for culturing the microorganism, or a combination thereof (in any order), for example, prior to culturing, but is not limited thereto.

The method for producing an L-amino acid of the present disclosure may further comprise recovering a desired substance, specifically an L-amino acid, from the cultured microorganism, a culture of the microorganism, a fermentation product of the microorganism, or the culture medium. The recovering may be further comprised after the culturing, but is not limited thereto.

The recovering may be collecting the desired L-amino acid using a suitable method known in the art, depending on the method for culturing the microorganism of the present disclosure, such as batch, continuous, or fed-batch culturing. For example, the recovery may involve centrifugation, filtration, treatment with a crystallized protein precipitating agent (salting-out method), extraction, sonication, ultrafiltration, dialysis, various kinds of chromatography such as molecular sieve chromatography (gel filtration), adsorption chromatography, ion exchange chromatography, and affinity chromatography, HPLC, or a combination thereof. A desired substance, specifically an L-amino acid, can be recovered from the medium or microorganism using a suitable method known in the art.

Additionally, the method for producing an L-amino acid of the present disclosure may further comprise purification. The purification may be performed using a suitable method known in the art. In one example, when the method for producing an L-amino acid of the present disclosure includes both the recovery and the purification, these steps may be performed continuously or discontinuously in any order, simultaneously, or as one integrated step, but are not limited thereto.

In the method of the present disclosure, the fructokinase derived from a microorganism of the genus *Escherichia*, the non-PTS sugar transporter derived from a microorganism of the genus *Zymomonas*, the polynucleotide encoding the same, the microorganism of the genus *Corynebacterium* comprising the same, and the L-amino acid, *etc.*, are as described in the other aspects above.

Still another aspect of the present disclosure provides a composition for producing an L-amino acid, comprising a microorganism of the genus *Corynebacterium* comprising: at least one selected from the group consisting of a fructokinase derived from a microorganism of the genus *Escherichia*, a polynucleotide encoding the same, a variant polypeptide of the fructokinase, and a polynucleotide encoding the variant polypeptide; and at least one selected from the group consisting of a non-PTS sugar transporter derived from a microorganism of the genus *Zymomonas*, and a polynucleotide encoding the same, the culture of the microorganism, the fermentation product of the microorganism, or a combination of two or more thereof.

The composition of the present disclosure may further comprise a suitable excipient commonly used in compositions for producing an L-amino acid. Examples of such excipients include preservatives, wetting agents, dispersing agents, suspending agents, buffering agents, stabilizers, or isotonic agents, but the excipient is not limited thereto.

In one specific embodiment, the composition of the present disclosure may comprise each component in a microbially effective amount or in an amount that can be suitably present in a composition for production.

In the composition of the present disclosure, the fructokinase derived from a microorganism of the genus *Escherichia*, the variant polypeptide and the non-PTS sugar transporter derived from a microorganism of the genus *Zymomonas*, the polynucleotide encoding the same, the microorganism of the genus *Corynebacterium* comprising the same, and the L-amino acid, *etc.*, are as described in the other aspects above.

Still another aspect of the present disclosure provides a use of a microorganism of the genus *Corynebacterium* comprising: at least one selected from the group consisting of a fructokinase derived from a microorganism of the genus *Escherichia*, a polynucleotide encoding the same, a variant polypeptide of the fructokinase, and a polynucleotide encoding the variant polypeptide; and at least one selected from the group consisting of a non-PTS sugar transporter derived from a microorganism of the genus *Zymomonas*, and a polynucleotide encoding the same, for producing an L-amino acid.

In the use of the present disclosure, the fructokinase derived from a microorganism of the genus *Escherichia*, the variant polypeptide and the non-PTS sugar transporter derived from a microorganism of the genus *Zymomonas*, the polynucleotide encoding the same, the microorganism of the genus *Corynebacterium* comprising the same, and the L-amino acid, *etc.*, are as described in the other aspects above.

### [Modes for Carrying Out the Invention]

Hereinafter, the present disclosure will be described in more detail by way of Examples and Experimental Examples. However, these Examples and Experimental Examples are given for illustrative purposes only, and the scope of the present disclosure is not intended to be limited by these Examples and Experimental Examples.

### Example 1. Tryptophan-producing strain construction

CA04-8357, a tryptophan-producing strain constructed in Korean Patent No. 10-2035844, was used. To enhance tryptophan-producing ability by increasing precursor biosynthesis, the *ptsF* gene encoding the phosphotransferase system responsible for fructose uptake was deleted. To construct a deletion vector, PCR was performed using the chromosomal DNA of *Corynebacterium glutamicum* ATCC 13869 as a template and primer pairs of SEQ ID NO: 1 and SEQ ID NO: 2, and SEQ ID NO: 3 and SEQ ID NO: 4. The polymerase used for PCR was Solg^{™} Pfu-X DNA polymerase (SolGent Co.), and the PCR amplification was carried out under the following conditions: denaturation at 95°C for 5 minutes, followed by 27 cycles of denaturation at 95°C for 20 seconds, annealing at 60°C for 40 seconds, and extension at 72°C for 1 minute, and a final extension at 72°C for 5 minutes.
SEQ ID NO: 1
   - tcgagctcggtacccATACCTCCGACAAGCCACTG
SEQ ID NO: 2
   - GATTGCCCAGACCACGAAGAATAGCAACTTGACCGGGGAC
SEQ ID NO: 3
   - GTCCCCGGTCAAGTTGCTATTCTTCGTGGTCTGGGCAATC
SEQ ID NO: 4
   - ctctagaggatccccAAAAGCAAAAGGCGGTACCA

As a result, DNA fragments of 719 bp and 738 bp were obtained, respectively. The obtained DNA product was purified using a PCR purification kit (QIAGEN), and the purified amplification product and the chromosomal transformation vector pDC24 (SEQ ID NO: 138) cleaved with SmaI restriction enzyme were cloned using the Gibson assembly method (DG Gibson et al., NATURE METHODS, VOL.6 NO.5, MAY 2009, NEBuilder HiFi DNA Assembly Master Mix) to obtain a recombinant plasmid, which was designated pDC24ΔptsF. The cloning was carried out by mixing the Gibson assembly reagent with each gene fragment at calculated molar ratios and incubating the mixture at 50°C for 1 hour. The constructed pDC24ΔptsF vector was transformed into the CA04-8357 strain by electroporation (Appl. Microbiol. Biotechnol. (1999) 52:541-545). After a second crossover process, the strain CA04-8357::ΔptsF, in which the *ptsF* gene was deleted on the chromosome, was obtained. The corresponding genetic manipulation was confirmed through PCR using primers of SEQ ID NO: 5 and SEQ ID NO: 6, which are capable of amplifying the external regions of the upstream and downstream homologous recombination sites where the corresponding gene has been deleted, respectively, and through genome sequencing.
SEQ ID NO: 5
   - CAGGTGGTAAAGGCATCAA
SEQ ID NO: 6
   - CTCTCGTTTGGTTGCTGT

The strain CA04-8357::ΔptsF thus obtained was designated *Corynebacterium glutamicum* CM05-9836.

### Example 2. Construction of Corynebacterium strains into which fructokinases of various microorganisms were introduced

### Example 2-1. Construction of plasmids for the insertion of fructokinases derived from various microorganisms

To insert a foreign fructokinase gene into the chromosome of *Corynebacterium glutamicum*, BBD29_02180-BBD29_02200, known as a gene encoding a transposon in *Corynebacterium glutamicum*, was used as the insertion site. Specifically, to construct vectors for BBD29_02180-BBD29_02200 deletion and target gene insertion, PCR was performed in the same manner as in Example 1 using the chromosome of ATCC13869 as a template and primer pairs of SEQ ID NO: 7 and SEQ ID NO: 8, and SEQ ID NO: 9 and SEQ ID NO: 10. Primers of SEQ ID NOs: 8 and 9 were designed to comprise a ScaI cleavage site between the left homologous arm and the right homologous arm.
SEQ ID NO: 7
   - AATTCGAGCTCGGTACCCAGTGGACACGGAGGATTT
SEQ ID NO: 8
   - TGGTGACCGCATTATGGagtactGTTTAGGGTGGGGATAA
SEQ ID NO: 9
   - TTATCCCCACCCTAAACagtactCCATAATGCGGTCACCA
SEQ ID NO: 10
   - GGTCGACTCTAGAGGATCCCCGTGTAAAGAGCAATCGGG

As a result, DNA fragments of 823 bp and 813 bp were obtained, respectively. The obtained DNA product was purified using a PCR purification kit, and the purified amplification product and the chromosomal transformation vector pDC24 cleaved with SmaI restriction enzyme were cloned using the Gibson assembly method to obtain a recombinant plasmid, which was designated pDC24ΔBBD29_02180-BBD29_02200. Gibson cloning was performed in the same manner as in Example 1.

### Example 2-2. Construction of a microorganism of Corynebacterium glutamicum into which the cscK gene derived from Escherichia coli was introduced

The *cscK* gene (SEQ ID NO: 11), a fructokinase derived from *Escherichia coli*, was introduced into the CM05-9836 strain constructed in Example 1 above. Information on the corresponding gene and the surrounding base sequences (CP002967.1) was obtained from the NIH GenBank. In order to amplify the *cscK* gene based on the obtained base sequence, PCR was performed in the same manner as in Example 1 using chromosomal DNA of the *Escherichia coli* strain as a template and primers of SEQ ID NO: 12 and SEQ ID NO: 13. As a result, a 950 bp DNA fragment comprising a 915 bp *cscK* gene was obtained.
SEQ ID NO: 12
   - CGAAAGGAAACACTCATGTCAGCCAAAGTATGGGT
SEQ ID NO: 13
   - TGGTGACCGCATTATGGagtCTATTCCAGTTCTTGTCGAC

To use the CJ7 promoter derived from *Corynebacterium stationis* (SEQ ID NO: 14, US 7,662,943 B2), PCR was performed in the same manner as in Example 1 using genomic DNA of *Corynebacterium stationis* as a template and primers of SEQ ID NO: 15 and SEQ ID NO: 16. As a result, a 353 bp DNA fragment comprising a 318 bp CJ7 promoter was obtained.
SEQ ID NO: 15
   - TTATCCCCACCCTAAACagtAGAAACATCCCAGCGCTACT
SEQ ID NO: 16
   - TACTTTGGCTGACATGAGTGTTTCCTTTCGTTGGG

After treating the pDC24ΔBBD29_02180-BBD29_02200 vector constructed in Example 2-1 with restriction enzyme ScaI, a recombinant plasmid was obtained by cloning the amplified CJ7 promoter region and the *cscK* gene fragment using the Gibson assembly method, and was designated pDC24ΔBBD29_02180-BBD29_02200:: Pcj7-csck. Gibson cloning was performed in the same manner as in Example 1. The constructed pDC24ΔBBD29_02180-BBD29_02200:: Pcj7-csck vector was transformed into the CM05-9836 strain constructed in Example 1 by electroporation. After a secondary crossover process, a strain into which 1 copy of the Pcj7-*cscK* gene was inserted was obtained. The corresponding genetic manipulation was confirmed through PCR using primers of SEQ ID NO: 17 and SEQ ID NO: 18, which are capable of amplifying the external regions of the upstream and downstream homologous recombination sites where the corresponding gene has been inserted, respectively, and through genome sequencing. The strain thus obtained was designated CM05-9837.
SEQ ID NO: 17
   - TTTTTCTCCCCTCGACCT
SEQ ID NO: 18
   - TCCTCCTTTCTTCTTCAAC

### Example 2-3. Construction of a microorganism of Corynebacterium glutamicum into which the mak gene derived from Escherichia coli was introduced

The mak gene (SEQ ID NO: 19), a fructokinase derived from *Escherichia coli*, was introduced into the strain constructed in Example 1 above. Information on the corresponding gene and the surrounding base sequences (NC_000913.3) was obtained from the NIH GenBank. In order to amplify the mak gene based on the obtained base sequence, PCR was performed in the same manner as in Example 1 using chromosomal DNA of the *Escherichia coli* strain as a template and primers of SEQ ID NO: 20 and SEQ ID NO: 21. As a result, a 944 bp DNA fragment comprising a 909 bp gene derived from *Escherichia coli* was obtained.
SEQ ID NO: 20
   - CGAAAGGAAACACTCGTGCGTATAGGTATCGATTTAGG
SEQ ID NO: 21
   - TGGTGACCGCATTATGGagtTTACTCTTGTGGCCATAACC

To use the CJ7 promoter derived from *Corynebacterium stationis* (SEQ ID NO: 14, US 7,662,943 B2), PCR was performed in the same manner as in Example 1 using genomic DNA of *Corynebacterium stationis* as a template and primers of SEQ ID NO: 15 and SEQ ID NO: 22.
SEQ ID NO: 22
- AAATCGATACCTATACGCACGAGTGTTTCCTTTCGTTGGG

After treating the pDC24ΔBBD29_02180-BBD29_02200 vector constructed in Example 2-1 with restriction enzyme ScaI, a recombinant plasmid was obtained by cloning the amplified CJ7 promoter region and the mak gene fragment using the Gibson assembly method, and was designated pDC24ΔBBD29_02180-BBD29_02200:: Pcj7-mak. Gibson cloning was performed in the same manner as in Example 1. The constructed pDC24ΔBBD29_02180-BBD29_02200:: Pcj7-mak vector was transformed into the CM05-9836 strain constructed in Example 1 by electroporation. After a secondary crossover process, a strain into which 1 copy of the Pcj7-mak gene was inserted was obtained. The corresponding genetic manipulation was confirmed through PCR using primers of SEQ ID NO: 17 and SEQ ID NO: 18, which are capable of amplifying the external regions of the upstream and downstream homologous recombination sites where the corresponding gene has been inserted, respectively, and through genome sequencing. The strain thus obtained was designated CM05-9838.

### Example 2-4. Construction of a microorganism of Corynebacterium glutamicum into which the scrK gene derived from Klebsiella pneumoniae was introduced

The *scrK* gene (SEQ ID NO: 23), a fructokinase derived from *Klebsiella pneumoniae*, was introduced into the strain constructed in Example 1 above. Information on the corresponding gene and the surrounding base sequences (NC_016845.1) was obtained from the NIH GenBank. In order to amplify the *scrK* gene based on the obtained base sequence, PCR was performed in the same manner as in Example 1 using chromosomal DNA of the *Klebsiella pneumoniae* strain as a template and primers of SEQ ID NO: 24 and SEQ ID NO: 25. As a result, a 959 bp DNA fragment comprising a 924 bp gene derived from *Klebsiella pneumoniae* was obtained.
SEQ ID NO: 24
   - AACGAAAGGAAACACTCATGAATGGAAAAATCTGGGT
SEQ ID NO: 25
   - GTGACCGCATTATGGagtTCACAGCGAGCGCTGAAGA

To use the CJ7 promoter derived from *Corynebacterium stationis* (SEQ ID NO: 14, US 7,662,943 B2), PCR was performed in the same manner as in Example 1 using genomic DNA of *Corynebacterium stationis* as a template and primers of SEQ ID NO: 15 and SEQ ID NO: 26.
SEQ ID NO: 26
- CCAGATTTTTCCATTCATGAGTGTTTCCTTTCGTTGGG

After treating the pDC24ΔBBD29_02180-BBD29_02200 vector constructed in Example 2-1 with restriction enzyme ScaI, a recombinant plasmid was obtained by cloning the amplified CJ7 promoter region and the *scrK* gene fragment using the Gibson assembly method, and was designated pDC24ΔBBD29_02180-BBD29_02200:: Pcj7-scrk. Gibson cloning was performed in the same manner as in Example 1. The constructed pDC24ΔBBD29_02180-BBD29_02200:: Pcj7-scrk vector was transformed into the CM05-9836 strain constructed in Example 1 by electroporation. After a secondary crossover process, a strain into which 1 copy of the Pcj7-scrk gene was inserted was obtained. The corresponding genetic manipulation was confirmed through PCR using primers of SEQ ID NO: 17 and SEQ ID NO: 18, which are capable of amplifying the external regions of the upstream and downstream homologous recombination sites where the corresponding gene has been inserted, respectively, and through genome sequencing. The strain thus obtained was designated CM05-9839.

### Example 2-5. Construction of a microorganism of Corynebacterium glutamicum into which the frk gene derived from Zymomonas mobilis was introduced

The *frk* gene (SEQ ID NO: 27), a fructokinase derived from *Zymomonas mobilis*, was introduced into the strain constructed in Example 1 above. Information on the corresponding gene and the surrounding base sequences (M97296.1) was obtained from the NIH GenBank. In order to amplify the *frk* gene based on the obtained base sequence, PCR was performed in the same manner as in Example 1 using chromosomal DNA of the *Zymomonas mobilis* strain as a template and primers of SEQ ID NO: 28 and SEQ ID NO: 29. As a result, a 936 bp DNA fragment comprising a 906 bp gene derived from *Zymomonas mobilis* was obtained.
SEQ ID NO: 28
   - CGAAAGGAAACACTCATGAAAAACGATAAAAAAATTTATGG
SEQ ID NO: 29
   - ACCGCATTATGGagtTTATTTATTTTCTGCAGCCAATG

To use the CJ7 promoter derived from *Corynebacterium stationis* (SEQ ID NO: 14, US 7,662,943 B2), PCR was performed in the same manner as in Example 1 using genomic DNA of *Corynebacterium stationis* as a template and primers of SEQ ID NO: 15 and SEQ ID NO: 30.
SEQ ID NO: 30
- TTTATCGTTTTTCATGAGTGTTTCCTTTCGTTGG

After treating the pDC24ΔBBD29_02180-BBD29_02200 vector constructed in Example 2-1 with restriction enzyme ScaI, a recombinant plasmid was obtained by cloning the amplified CJ7 promoter region and the *frk* gene fragment using the Gibson assembly method, and was designated pDC24ΔBBD29_02180-BBD29_02200:: PCJ7-frk. Gibson cloning was performed in the same manner as in Example 1. The constructed pDC24ΔBBD29_02180-BBD29_02200:: Pcj7-frk vector was transformed into the CM05-9836 strain constructed in Example 1 by electroporation. After a secondary crossover process, a strain into which 1 copy of the Pcj7-frk gene was inserted was obtained. The corresponding genetic manipulation was confirmed through PCR using primers of SEQ ID NO: 17 and SEQ ID NO: 18, which are capable of amplifying the external regions of the upstream and downstream homologous recombination sites where the corresponding gene has been inserted, respectively, and through genome sequencing. The strain thus obtained was designated CM05-9840.

### Example 3. Evaluation of sugar utilization/L-tryptophan-producing ability of microorganisms of the genus Corynebacterium, into which fructokinases derived from various microorganisms were introduced

To confirm the L-tryptophan-producing ability and fructose utilization of the strains constructed in the above examples, the strains were cultured and evaluated using the following method. Each strain was inoculated into a 250-mL corner-baffled flask containing 25 mL of seed medium and incubated with shaking at 200 rpm at 30°C for 20 hours. Then, 1 mL of the seed culture was inoculated into a 250-mL corner-baffled flask containing 25 mL of production medium and incubated with shaking at 200 rpm at 30°C for 30 hours. After completion of the incubation, L-tryptophan production was measured by HPLC.

### [Seed Medium (pH 7.0)]

Glucose 20 g, Peptone 10 g, Yeast extract 5 g, Urea 1.5 g, KH₂PO₄ 4 g, K₂HPO₄ 8 g, MgSO₄·7H₂O 0.5 g, Biotin 100 µg, Thiamine HCl 1000 µg, Calcium pantothenate 2000 µg, and Nicotinamide 2000 µg (per 1 L of distilled water).

### [Production Medium (pH 7.0)]

Fructose 30 g, (NH₄)₂SO₄ 15 g, MgSO₄·7H₂O 1.2 g, KH₂PO₄ 1 g, Yeast extract 5 g, Biotin 900 µg, Thiamine HCl 4500 µg, Calcium pantothenate 4500 µg, and CaCO₃ 30 µg (per 1 L of distilled water).

**[Table 1]**

| Comparison of fructose utilization and L-tryptophan-producing ability of L-tryptophan-producing strains derived from *Corynebacterium glutamicum* ATCC 13869 (30 hr) | | |
|---|---|---|
| Strain No. | Fructose consumed (g/L) | L-tryptophan production (g/L) |
| CA04-8357 | 30 | 2.1 |
| CM05-9836 | 1.2 | 0.05 |
| CM05-9837 | 3.2 | 0.3 |
| CM05-9838 | 1.2 | 0.03 |
| CM05-9839 | 1.4 | 0.04 |
| CM05-9840 | 8.6 | 0.8 |

In addition to the parent strain CA04-8357, which has L-tryptophan-producing ability, CM05-9836, CM05-9837, CM05-9838, CM05-9839, and CM05-9840 were cultured under conditions using fructose as the sole carbon source. In addition, the concentration of L-tryptophan and sugar consumption in the culture were measured, and the results are shown in Table 1 above.

In the case of CA04-8357, in which *ptsF* was present, it consumed the added fructose completely within a given time and produced 2.1 g/L of L-tryptophan. However, in the case of CM05-9836, in which *ptsF* was deleted from CA04-8357, it barely consumed the sugar and produced a significantly lower concentration of tryptophan compared to the parent strain CA04-8357.

CM05-9838 and CM05-9839, into which mak and *scrK* were introduced, showed levels of sugar consumption ability and L-tryptophan-producing ability similar to those of CM05-9836, confirming that mak and *scrK* have almost no effect as fructokinases.

On the other hand, in the case of CM05-9837, into which *cscK* was introduced, sugar consumption and tryptophan production increased slightly compared to those of the parent strain CA04-8357. In the case of CM05-9840, into which *frk* was introduced, it showed the highest level of sugar consumption ability and produced 0.8 g/L of L-tryptophan.

From the above results, it was confirmed that, under conditions of using fructose as the sole carbon source, *frk* exhibits the most excellent function as a fructokinase.

However, when culturing microorganisms in general, culturing in the presence of both fructose and glucose is more common than using fructose as the sole carbon source. Therefore, the same strains evaluated in the present Example were cultured and evaluated in a fructose/glucose mixed medium using the following method. Each strain was inoculated into a 250-mL corner-baffled flask containing 25 mL of seed medium and incubated with shaking at 200 rpm at 30°C for 20 hours. Then, 1 mL of the seed culture was inoculated into a 250-mL corner-baffled flask containing 25 mL of mixed medium and incubated with shaking at 200 rpm at 30°C for 20 hours. After completion of the incubation, L-tryptophan production was measured by HPLC.

### [Seed Medium (pH 7.0)]

Glucose 20 g, Peptone 10 g, Yeast extract 5 g, Urea 1.5 g, KH₂PO₄ 4 g, K₂HPO₄ 8 g, MgSO₄·7H₂O 0.5 g, Biotin 100 µg, Thiamine HCl 1000 µg, Calcium pantothenate 2000 µg, and Nicotinamide 2000 µg (per 1 L of distilled water).

### [Fructose/glucose mixed medium (pH 7.0)]

Fructose 15 g, Glucose 15 g, (NH₄)₂SO₄ 15 g, MgSO₄·7H₂O 1.2 g, KH₂PO₄ 1 g, Yeast extract 5 g, Biotin 900 µg, Thiamine HCl 4500 µg, Calcium pantothenate 4500 µg, and CaCO₃ 30 µg (per 1 L of distilled water).

**[Table 2]**

| Comparison of glucose and fructose availability and L-tryptophan-producing ability of L-tryptophan-producing strains derived from *Corynebacterium glutamicum* ATCC 13869 (20 hr) | | | |
|---|---|---|---|
| Strain No. | Glucose consumed (g/L) | Fructose consumed (g/L) | L-tryptophan production (g/L) |
| CA04-8357 | 11.7 | 15 | 1.8 |
| CM05-9836 | 13.9 | 0.2 | 0.7 |
| CM05-9837 | 14.3 | 2.3 | 1.1 |
| CM05-9838 | 14.1 | 0.5 | 0.8 |
| CM05-9839 | 14 | 0.3 | 0.8 |
| CM05-9840 | 13.8 | 1.1 | 0.9 |

It was confirmed that all five strains constructed under the present evaluation conditions consumed glucose at a level similar to that of the parent strain CA04-8357. On the other hand, the fructose consumption rate showed different results compared to the conditions using fructose as the sole carbon source. The *frk*-introduced strain CM05-9840, which exhibited the most excellent sugar consumption rate under the conditions of using fructose as the sole carbon source, showed a lower sugar consumption rate in the fructose/glucose mixed medium compared to the *cscK-*introduced strain CM05-9837. Therefore, it was determined that *cscK*, which exhibited an appropriate level of activity under conditions of using fructose as the sole carbon source without inhibition of activity by glucose, is the most effective factor as a fructokinase.

### Example 4. Construction of microorganisms of the genus Corynebacterium, into which non-PTS sugar transporters of various microorganisms were introduced

### Example 4-1. Screening and selection of non-PTS fructose uptake genes

The tryptophan-producing ability was to be further improved by increasing fructose uptake in the strains.

Foreign genes known to be capable of introducing sugar were searched, and 14 candidate genes were selected. As a result, taking into consideration the biosafety level applicable to production strains and the feasibility of securing the organisms, seven organisms were selected, as shown in Table 3 below.

**[Table 3]**

| Order | Strain | Genome Accession Number | Biosafety | Gene sequence |
|---|---|---|---|---|
| 1 | *Zymomonas mobilis* ATCC10988 | CP002850.1 | 1 | 35 |
| 2 | *Corynebacterium glutamicum* ATCC13869 | CP016335.1 | 1 | 42 |
| 3 | *Zygosaccharomyces bailii* | AJ515522.1 | 1 | 46 |
| 4 | *Kluyveromyces lactis* | NC_006041.1 | 1 | 50 |
| 5 | *Saccharomyces cerevisiae* | NC_001136.10 | 1 | 54 |
| 6 | *Zygosaccharomyces rouxii* | XM_002495633.1 | 1 | 58 |
| 7 | *Saccharomyces pastorianus* | AJ250992.1 | 1 | 62 |

### Example 4-2. Construction of plasmids for the insertion of non-PTS sugar transporters derived from various microorganisms

To insert a foreign non-PTS sugar transporter into the chromosome of *Corynebacterium glutamicum*, BBD29_12045-BBD29_12055, known as a gene encoding a transposon in *Corynebacterium glutamicum*, was used as the insertion site. Specifically, to construct vectors for BBD29_12045-BBD29_12055 deletion and target gene insertion, PCR was performed in the same manner as in Example 1 using the chromosome of ATCC13869 as a template and primer pairs of SEQ ID NO: 31 and SEQ ID NO: 32, and SEQ ID NO: 33 and SEQ ID NO: 34. Primers of SEQ ID NOs: 32 and 33 were designed to comprise a ScaI cleavage site between the left homologous arm and the right homologous arm.
SEQ ID NO: 31
   - AATTCGAGCTCGGTACCCGATGGAACTACGAGACT
SEQ ID NO: 32
   - TGACAATCACCGCATCCagtactGGATATTCGAGACAG
SEQ ID NO: 33
   - CTGTCTCGAATATCCagtactGGATGCGGTGATTGTCAG
SEQ ID NO: 34
   - GGTCGACTCTAGAGGATCCCCTAACCACGACGAC

As a result, DNA fragments of 793 bp and 843 bp were obtained, respectively. The obtained DNA product was purified using a PCR purification kit, and the purified amplification product and the chromosomal transformation vector pDC24 cleaved with SmaI restriction enzyme were cloned using the Gibson assembly method to obtain a recombinant plasmid, which was designated pDC24ΔBBD29_12045-BBD29_12055. Gibson cloning was performed in the same manner as in Example 1.

### Example 4-3. Construction of a microorganism of the genus Corynebacterium, into which the glf gene derived from Zymomonas mobilis was introduced

In order to enhance the fructose uptake ability of the CM05-9837 strain constructed in Example 2-2 above, the *glf* gene (SEQ ID NO: 35) encoding a non-PTS sugar uptake protein derived from *Zymomonas mobilis* selected in Example 4-1 was introduced. Information on the corresponding gene and the surrounding base sequences (CP002850.1) was obtained from the NIH GenBank. In order to amplify the *glf* gene based on the obtained base sequence, PCR was performed in the same manner as in Example 1 using chromosomal DNA of the *Zymomonas mobilis* strain as a template and primers of SEQ ID NO: 36 and SEQ ID NO: 37. As a result, a 1452 bp DNA fragment comprising a 1422 bp *glf* gene was obtained.
SEQ ID NO: 36
   - CGAAAGGAAACACTCATGAGTTCTGAAAGTAGTCA
SEQ ID NO: 37
   - ATCACCGCATCCAGTCTACTTCTGGGAGCGCCACA

To use the CJ7 promoter derived from *Corynebacterium stationis* (SEQ ID NO: 14, US 7,662,943 B2), PCR was performed in the same manner as in Example 1 using genomic DNA of *Corynebacterium stationis* as a template and primers of SEQ ID NO: 38 and SEQ ID NO: 39.
SEQ ID NO: 38
   - TCTCGAATATCCAGTAGAAACATCCCAGCGCTACT
SEQ ID NO: 39
   - ACTTTCAGAACTCATGAGTGTTTCCTTTCGTTGGG

After treating the pDC24ΔBBD29_12045-BBD29_12055 vector constructed in Example 4-2 with restriction enzyme ScaI, a recombinant plasmid was obtained by cloning the amplified CJ7 promoter region and the *glf* gene fragment using the Gibson assembly method, and was designated pDC24ΔBBD29_12045-BBD29_12055:: Pcj7-glf. Gibson cloning was performed in the same manner as in Example 1. The constructed pDC24-Pcj7-glf vector was transformed into the CM05-9837 strain constructed in Example 2-2 by electroporation. After a secondary crossover process, a strain into which 1 copy of the Pcj7-glf gene was inserted was obtained. The corresponding genetic manipulation was confirmed through PCR using primers of SEQ ID NO: 40 and SEQ ID NO: 41, which are capable of amplifying the external regions of the upstream and downstream homologous recombination sites where the corresponding gene has been inserted, respectively, and through genome sequencing. The strain thus obtained was designated CM05-9841.
SEQ ID NO: 40
   - AACAACACCACATCTACATC
SEQ ID NO: 41
   - CAGCCTTTTCCAGCACCA

### Example 4-4. Construction of a microorganism of the genus Corynebacterium, into which the iolT1 gene derived from Corynebacterium glutamicum was introduced

In order to enhance the fructose uptake ability of the CM05-9837 strain constructed in Example 2-2 above, the *iolT1* gene (SEQ ID NO: 42) encoding a non-PTS sugar uptake protein derived from *Corynebacterium glutamicum* selected in Example 4-1 was introduced. Information on the corresponding gene and the surrounding base sequences (CP016335.1) was obtained from the NIH GenBank. In order to amplify the *iolT1* gene based on the obtained base sequence, PCR was performed in the same manner as in Example 1 using chromosomal DNA of the *Corynebacterium glutamicum* strain as a template and primers of SEQ ID NO: 43 and SEQ ID NO: 44. As a result, a 1506 bp DNA fragment comprising a 1476 bp *iolT1* gene was obtained.
SEQ ID NO: 43
   - CGAAAGGAAACACTCATGGCTAGTACCTTCATTCAGGC
SEQ ID NO: 44
   - ATCACCGCATCCAGTTTAGTGCACCTTTCCTTTTCG

To use the CJ7 promoter derived from *Corynebacterium stationis* (SEQ ID NO: 14, US 7,662,943 B2), PCR was performed in the same manner as in Example 1 using genomic DNA of *Corynebacterium stationis* as a template and primers of SEQ ID NO: 38 and SEQ ID NO: 45.
SEQ ID NO: 45
- GAAGGTACTAGCCATGAGTGTTTCCTTTCGTTGGG

After treating the pDC24ΔBBD29_12045-BBD29_12055 vector constructed in Example 4-2 with the restriction enzyme ScaI, a recombinant plasmid was obtained by cloning the amplified CJ7 promoter region and *iolT1* gene fragment using the Gibson assembly method, and was designated pDC24ΔBBD29_12045-BBD29_12055:: Pcj7-iolT1. Gibson cloning was performed in the same manner as in Example 1. The constructed pDC24-Pcj7-iolT1 vector was transformed into the CM05-9837 strain constructed in Example 2-2 by electroporation. After a secondary crossover process, a strain into which 1 copy of the Pcj7-iolT1 gene was inserted was obtained. The corresponding genetic manipulation was confirmed through PCR using primers of SEQ ID NO: 40 and SEQ ID NO: 41, which are capable of amplifying the external regions of the upstream and downstream homologous recombination sites where the corresponding gene has been inserted, respectively, and through genome sequencing. The strain thus obtained was designated CM05-9842.

### Example 4-5. Construction of a microorganism of the genus Corynebacterium, into which the ffz1 gene derived from Zygosaccharomyces bailii was introduced

In order to enhance the fructose uptake ability of the CM05-9837 strain constructed in Example 2-2 above, the *ffz1* gene (SEQ ID NO: 46) encoding a non-PTS sugar uptake protein derived from Zygosaccharomyces bailii selected in Example 4-1 was introduced. Information on the gene encoding a membrane protein and the surrounding base sequences (AJ515522.1) was obtained from the NIH GenBank. In order to amplify the *ffz1* gene based on the obtained base sequence, PCR was performed in the same manner as in Example 1 using chromosomal DNA of the Zygosaccharomyces bailii strain as a template and primers of SEQ ID NO: 47 and SEQ ID NO: 48. As a result, a 1884 bp DNA fragment comprising the 1854 bp *ffz1* gene was obtained.
SEQ ID NO: 47
   - CGAAAGGAAACACTCATGGTTAAGATAGACGCTTC
SEQ ID NO: 48
   - ACAATCACCGCATCCAGTTTATTCATCATCATGCT

To use the CJ7 promoter derived from *Corynebacterium stationis* (SEQ ID NO: 14, US 7,662,943 B2), PCR was performed in the same manner as in Example 1 using genomic DNA of *Corynebacterium stationis* as a template and primers of SEQ ID NO: 38 and SEQ ID NO: 49.
SEQ ID NO: 49
- GTCTATCTTAACCATGAGTGTTTCCTTTCGTTGGG

After treating the pDC24ΔBBD29_12045-BBD29_12055 vector constructed in Example 4-2 with restriction enzyme ScaI, a recombinant plasmid was obtained by cloning the amplified CJ7 promoter region and *ffz1* gene fragment using the Gibson assembly method, and was designated pDC24ΔBBD29_12045-BBD29_12055:: Pcj7-ffz1. Gibson cloning was performed in the same manner as in Example 1. The constructed pDC24ΔBBD29_12045-BBD29_12055:: Pcj7- ffz1 vector was transformed into the CM05-9837 strain constructed in Example 2-2 by electroporation. After a secondary crossover process, a strain into which 1 copy of the Pcj7-ffz1 gene was inserted was obtained. The corresponding genetic manipulation was confirmed through PCR using primers of SEQ ID NO: 40 and SEQ ID NO: 41, which are capable of amplifying the external regions of the upstream and downstream homologous recombination sites where the corresponding gene has been inserted, respectively, and through genome sequencing. The strain thus obtained was designated CM05-9843.

### Example 4-6. Construction of a microorganism of the genus Corynebacterium, into which the frt1 gene derived from Kluyveromyces lactis was introduced

In order to enhance the fructose uptake ability of the CM05-9837 strain constructed in Example 2-2 above, the *frt1* gene (SEQ ID NO: 50) encoding a non-PTS sugar uptake protein derived from *Kluyveromyces lactis* selected in Example 4-1 was introduced. Information on the gene encoding a membrane protein and the surrounding base sequences (NC_006041.1) was obtained from the NIH GenBank. In order to amplify the *frt1* gene based on the obtained base sequence, PCR was performed in the same manner as in Example 1 using chromosomal DNA of the *Kluyveromyces lactis* strain as a template and primers of SEQ ID NO: 51 and SEQ ID NO: 52. As a result, a 1731 bp DNA fragment comprising a 1701 bp *frt1* gene was obtained.
SEQ ID NO: 51
   - CGAAAGGAAACACTCATGTCTAGTAATCTGTCTGA
SEQ ID NO: 52
   - ATCACCGCATCCAGTTTAAATAGATTTACGGTTAC

To use the CJ7 promoter derived from *Corynebacterium stationis* (SEQ ID NO: 14, US 7,662,943 B2), PCR was performed in the same manner as in Example 1 using genomic DNA of *Corynebacterium stationis* as a template and primers of SEQ ID NO: 38 and SEQ ID NO: 53.
SEQ ID NO: 53
- CAGATTACTAGACATGAGTGTTTCCTTTCGTTGGG

After treating the pDC24ΔBBD29_12045-BBD29_12055 vector constructed in Example 4-2 with the restriction enzyme ScaI, a recombinant plasmid was obtained by cloning the amplified CJ7 promoter region and the *frt1* gene fragment using the Gibson assembly method, and was designated pDC24ΔBBD29_12045-BBD29_12055:: Pcj7-frt1. Gibson cloning was performed in the same manner as in Example 1. The constructed pDC24ΔBBD29_12045-BBD29_12055:: Pcj7-frt1 vector was transformed into the CM05-9837 strain constructed in Example 2-2 by electroporation. After a secondary crossover process, a strain into which 1 copy of the Pcj7-frt1 gene was inserted was obtained. The corresponding genetic manipulation was confirmed through PCR using primers of SEQ ID NO: 40 and SEQ ID NO: 41, which are capable of amplifying the external regions of the upstream and downstream homologous recombination sites where the corresponding gene has been inserted, respectively, and through genome sequencing. The strain thus obtained was designated CM05-9844.

### Example 4-7. Construction of a microorganism of the genus Corynebacterium, into which the hxt6 gene derived from Saccharomyces cerevisiae was introduced

In order to enhance the fructose uptake ability of the CM05-9837 strain constructed in Example 2-2 above, the *hxt6* gene (SEQ ID NO: 54) encoding a non-PTS sugar uptake protein derived from *Saccharomyces cerevisiae* selected in Example 4-1 was introduced. Information on the gene encoding a membrane protein and the surrounding base sequences (NC_001136.10) was obtained from the NIH GenBank. In order to amplify the *hxt6* gene based on the obtained base sequence, PCR was performed in the same manner as in Example 1 using chromosomal DNA of the *Saccharomyces cerevisiae* strain as a template and primers of SEQ ID NO: 55 and SEQ ID NO: 56. As a result, a 1743 bp DNA fragment comprising a 1713 bp *hxt6* gene was obtained.
SEQ ID NO: 55
   - CGAAAGGAAACACTCATGTCACAAGACGCTGCTAT
SEQ ID NO: 56
   - ATCACCGCATCCAGTTTATTTGGTGCTGAACATTC

To use the CJ7 promoter derived from *Corynebacterium stationis* (SEQ ID NO: 14, US 7,662,943 B2), PCR was performed in the same manner as in Example 1 using genomic DNA of *Corynebacterium stationis* as a template and primers of SEQ ID NO: 38 and SEQ ID NO: 57.
SEQ ID NO: 57
- AGCGTCTTGTGACATGAGTGTTTCCTTTCGTTGGG

After treating the pDC24ΔBBD29_12045-BBD29_12055 vector constructed in Example 4-2 with restriction enzyme ScaI, a recombinant plasmid was obtained by cloning the amplified CJ7 promoter region and the *hxt6* gene fragment using the Gibson assembly method, and was designated pDC24ΔBBD29_12045-BBD29_12055:: Pcj7-hxt6. Gibson cloning was performed in the same manner as in Example 1. The constructed pDC24ΔBBD29_12045-BBD29_12055:: Pcj7-hxt6 vector was transformed into the CM05-9837 strain constructed in Example 2-1 by electroporation. After a secondary crossover process, a strain into which 1 copy of the Pcj7-hxt6 gene was inserted was obtained. The corresponding genetic manipulation was confirmed through PCR using primers of SEQ ID NO: 40 and SEQ ID NO: 41, which are capable of amplifying the external regions of the upstream and downstream homologous recombination sites where the corresponding gene has been inserted, respectively, and through genome sequencing. The strain thus obtained was designated CM05-9845.

### Example 4-8. Construction of a microorganism of the genus Corynebacterium, into which the fsy1 gene derived from Zygosaccharomyces rouxii was introduced

In order to enhance the fructose uptake ability of the CM05-9837 strain constructed in Example 2-2 above, the fsy1(zro) gene (SEQ ID NO: 58) encoding a non-PTS sugar uptake protein derived from *Zygosaccharomyces rouxii* selected in Example 4-1 was introduced. Information on the gene encoding a membrane protein and the surrounding base sequences (XM_002495633.1) was obtained from the NIH GenBank. In order to amplify the fsy1(zro) gene based on the obtained base sequence, PCR was performed in the same manner as in Example 1 using chromosomal DNA of the *Saccharomyces cerevisiae* strain as a template and primers of SEQ ID NO: 59 and SEQ ID NO: 60. As a result, a 1725 bp DNA fragment comprising a 1695 bp fsy1(zro) gene was obtained.
SEQ ID NO: 59
   - CGAAAGGAAACACTCATGAAGTTTTCTACTTGGCG
SEQ ID NO: 60
   - ATCACCGCATCCAGTCTAATAGCTTAGTTTACCTC

To use the CJ7 promoter derived from *Corynebacterium stationis* (SEQ ID NO: 14, US 7,662,943 B2), PCR was performed in the same manner as in Example 1 using genomic DNA of *Corynebacterium stationis* as a template and primers of SEQ ID NO: 38 and SEQ ID NO: 61.
SEQ ID NO: 61
- AGTAGAAAACTTCATGAGTGTTTCCTTTCGTTGGG

After treating the pDC24ΔBBD29_12045-BBD29_12055 vector constructed in Example 4-2 with restriction enzyme ScaI, a recombinant plasmid was obtained by cloning the amplified CJ7 promoter region and the fsy1(zro) gene fragment using the Gibson assembly method, and was designated pDC24ΔBBD29_12045-BBD29_12055:: Pcj7-fsy1(zro). Gibson cloning was performed in the same manner as in Example 1. The constructed pDC24ΔBBD29_12045-BBD29_12055:: Pcj7-fsy1(zro) vector was transformed into the CM05-9837 strain constructed in Example 2-2 by electroporation. After a secondary crossover process, a strain into which 1 copy of the Pcj7-fsy1(zro) gene was inserted was obtained. The corresponding genetic manipulation was confirmed through PCR using primers of SEQ ID NO: 40 and SEQ ID NO: 41, which are capable of amplifying the external regions of the upstream and downstream homologous recombination sites where the corresponding gene has been inserted, respectively, and through genome sequencing. The strain thus obtained was designated CM05-9846.

### Example 4-9. Construction of a microorganism of the genus Corynebacterium, into which the fsy1 gene derived from Saccharomyces pastorianus was introduced

In order to enhance the fructose uptake ability of the CM05-9837 strain constructed in Example 2-2 above, the fsy1(spa) gene (SEQ ID NO: 62) encoding a non-PTS sugar uptake protein derived from *Saccharomyces pastorianus* selected in Example 4-1 was introduced. Information on the gene encoding a membrane protein and the surrounding base sequences (AJ250992.1) was obtained from the NIH GenBank. In order to amplify the fsy1(spa) gene based on the obtained base sequence, PCR was performed in the same manner as in Example 1 using chromosomal DNA of the *Saccharomyces pastorianus* strain as a template and primers of SEQ ID NO: 63 and SEQ ID NO: 64. As a result, a 1743 bp DNA fragment comprising a 1713 bp fsy1(spa) gene was obtained.
SEQ ID NO: 63
   - CGAAAGGAAACACTCATGTCACATGTTAACGCGTC
SEQ ID NO: 64
   - ATCACCGCATCCAGTCTAATAGCTCAATTGGCCCT

To use the CJ7 promoter derived from *Corynebacterium stationis* (SEQ ID NO: 14, US 7,662,943 B2), PCR was performed in the same manner as in Example 1 using genomic DNA of *Corynebacterium stationis* as a template and primers of SEQ ID NO: 38 and SEQ ID NO: 65.
SEQ ID NO: 65
- GTTAACATGTGACATGAGTGTTTCCTTTCGTTGGG

After treating the pDC24ΔBBD29_12045-BBD29_12055 vector constructed in Example 4-2 with restriction enzyme ScaI, a recombinant plasmid was obtained by cloning the amplified CJ7 promoter region and the fsy1(spa) gene fragment using the Gibson assembly method, and was designated pDC24ΔBBD29_12045-BBD29_12055:: Pcj7-fsy1(spa). Gibson cloning was performed in the same manner as in Example 1. The constructed pDC24ΔBBD29_12045-BBD29_12055:: Pcj7-fsy1(spa) vector was transformed into the CM05-9837 strain constructed in Example 2-2 by electroporation. After a secondary crossover process, a strain into which 1 copy of the Pcj7-fsy1(spa) gene was inserted was obtained. The corresponding genetic manipulation was confirmed through PCR using primers of SEQ ID NO: 40 and SEQ ID NO: 41, which are capable of amplifying the external regions of the upstream and downstream homologous recombination sites where the corresponding gene has been inserted, respectively, and through genome sequencing. The strain thus obtained was designated CM05-9847.

### Example 5. Evaluation of sugar utilization/L-tryptophan-producing ability of microorganisms of the genus Corynebacterium, into which non-PTS sugar transporters derived from various microorganisms were introduced

To confirm the L-tryptophan-producing ability and fructose utilization of the strains constructed in Example 4, the strains were cultured and evaluated using the following method. Each strain was inoculated into a 250-mL corner-baffled flask containing 25 mL of seed medium and incubated with shaking at 200 rpm at 30°C for 20 hours. Then, 1 mL of the seed culture was inoculated into a 250-mL corner-baffled flask containing 25 mL of mixed medium and incubated with shaking at 200 rpm at 30°C for 20 hours. After completion of the incubation, L-tryptophan production was measured by HPLC.

### < Seed medium (pH 7.0) >

Glucose 20 g, Peptone 10 g, Yeast extract 5 g, Urea 1.5 g, KH₂PO₄ 4 g, K₂HPO₄ 8 g, MgSO₄·7H₂O 0.5 g, Biotin 100 µg, Thiamine HCl 1000 µg, Calcium pantothenate 2000 µg, and Nicotinamide 2000 µg (per 1 L of distilled water).

### [Fructose/glucose mixed medium (pH 7.0)]

Fructose 15 g, Glucose 15 g, (NH₄)₂SO₄ 15 g, MgSO₄·7H₂O 1.2 g, KH₂PO₄ 1 g, Yeast extract 5 g, Biotin 900 µg, Thiamine HCl 4500 µg, Calcium pantothenate 4500 µg, and CaCO₃ 30 µg (per 1 L of distilled water).

**[Table 4]**

| Comparison of glucose and fructose utilization and L-tryptophan-producing ability of L-tryptophan-producing strains derived from *Corynebacterium glutamicum* ATCC 13869, into which a fructose utilization factor was introduced (20 hr) | | | | |
|---|---|---|---|---|
| Strain No. | Glucose consumed (g/L) | Fructose consumed (g/L) | Total amount of sugar consumed (g/L) | L-tryptophan production (g/L) |
| CM05-9837 | 14.1 | 2.2 | 16.3 | 1.0 |
| CM05-9841 | 12.3 | 10.3 | 22.5 | 2.2 |
| CM05-9842 | 13.5 | 3.4 | 16.9 | 1.1 |
| CM05-9843 | 13.5 | 4.2 | 17.7 | 1.3 |
| CM05-9844 | 13.2 | 3 | 16.2 | 1.2 |
| CM05-9845 | 7.3 | 6.9 | 14.2 | 0.6 |
| CM05-9846 | 9.4 | 7.4 | 16.8 | 1 |
| CM05-9847 | 7.5 | 6.9 | 14.4 | 0.9 |

Based on the strains constructed in Example 4, culturing was carried out in a glucose and fructose mixed medium. As a result, as shown in Table 4 above, it was confirmed that the parent strain CM05-9837 consumed most of the glucose, but had a low fructose consumption ability. However, the CM05-9841 strain, into which a non-PTS sugar uptake protein derived from *Zymomonas mobilis* was introduced, showed a fructose consumption ability approximately 4.7 times higher than that of the parent strain and exhibited the highest tryptophan productivity. Through the above results, it was confirmed that among a total of 7 types of foreign non-PTS fructose uptake factors, *glf* derived from *Zymomonas mobilis* was the most effective, and when combined with *cscK*, it increased tryptophan production along with the greatest improvement in sugar uptake ability.

### Example 6. Evaluation of sugar utilization/L-histidine-producing ability of microorganisms of the genus Corynebacterium, into which the cscK gene derived from a microorganism of the genus Escherichia and the glf gene derived from a microorganism of the genus Zymomonas were introduced

### Example 6-1. Construction of histidine-producing strains

To evaluate L-histidine-producing ability, the *Corynebacterium glutamicum* strain CJH1 was constructed.

Specifically, to resolve the feedback inhibition of the HisG protein, the first enzyme of the L-histidine biosynthetic pathway, the amino acids at positions 233 and 235 from the N-terminus of HisG were simultaneously substituted from glycine to histidine and from threonine to glutamine, respectively (SEQ ID NO: 66) (ACS Synth. Biol., 2014, 3 (1), pp 21-29). In addition, to enhance the activity of the hisE gene present in operons such as hisG, the start codon was substituted from GTG to ATG. Additionally, to enhance the L-histidine biosynthetic pathway, the promoters of the biosynthetic genes hisN, hisH, hisD, hisA, and hisB were replaced with strong promoters, and additional copies of the hisE(g1a)G(G233H/T235Q) gene and the hisD gene were introduced to enhance the corresponding pathway.

### Example 6-1-1. Production of a histidine-producing strain with resolved feedback inhibition

To construct a histidine-producing strain with relieved feedback inhibition, PCR was performed in the same manner as in Example 1 using chromosomal DNA of *Corynebacterium glutamicum* ATCC 13032. The left homologous arm fragment of 'hisE(g1a)G(G233H/T235Q)' was obtained using primers of SEQ ID NO: 67 and SEQ ID NO: 68, and the right homologous arm fragment of 'hisE(g1a)G(G233H/T235Q)' was obtained using primers of SEQ ID NO: 69 and SEQ ID NO: 70. PCR was performed in the same manner as in Example 1 using the two amplified DNA fragments above as templates and primers of SEQ ID NO: 67 and SEQ ID NO: 70 to obtain the 'hisE(g1a)G(G233H/T235Q)' gene fragment. In addition, PCR was performed using primers of SEQ ID NOs: 71 and 72 with ATCC13032 chromosomal DNA as a template, and the upstream region of the hisE gene was obtained.
SEQ ID NO: 67
   - gaggagatcaaaacaATGAAGACATTTGAC
SEQ ID NO: 68
   - AGTGGGGATACCTGTGGGTGGGATAAGCCT
SEQ ID NO: 69
   - GGCTTATCCCACCCACAGGTATCCCCACTG
SEQ ID NO: 70
   - ACTCTAGAGGATCCCCCTAGATGCGGGC
SEQ ID NO: 71
   - TCGAGCTCGGTACCCACCGAACTCCTGACAGAGT
SEQ ID NO: 72
   - acatgaagcgccTCGGTACATTCTTCCACA

In order to replace the promoter with a strong promoter, PCR was performed in the same manner as in Example 1 using the synthetic Pspl13 promoter (SEQ ID NO: 73, US 10,584,338 B2) as a template and primers of SEQ ID NO: 74 and SEQ ID NO: 75.
SEQ ID NO: 74
   - AAGAATGTACCGAggcgcttcatgtcaaca
SEQ ID NO: 75
   - CAAATGTCTTCATtgttttgatctcctcca

After treating the pDC24 vector with restriction enzyme SmaI, a recombinant plasmid was obtained by cloning the amplified upstream DNA fragment of the *hisE* gene, the Pspl13 promoter region, and the hisEG(G233H/T235Q) gene fragment using the Gibson assembly method, and was designated pDC24ΔPn_hisEG:: Pspl13_hisEG(G233H/T235Q). Gibson cloning was performed in the same manner as in Example 1. The constructed pDC24ΔPn_hisEG::Pspl13_hisEG(G233H/T235Q) vector was transformed into *Corynebacterium glutamicum* ATCC 13032 by electroporation. After a second crossover process, a strain was obtained in which feedback inhibition was relieved by introducing mutations into the native *hisE* gene, and hisE activity was enhanced by replacing the start codon of the *hisE* gene. The genetic manipulation was confirmed through PCR using primers of SEQ ID NO: 76 and SEQ ID NO: 77 and genome sequencing. The strain thus obtained was designated CJ-HIS1.
SEQ ID NO: 76
   - AGCTTTTCGACGAATCCC
SEQ ID NO: 77
   - CTGCCTCTCACAAGTTGAAG

### Example 6-1-2. Production of histidine-producing strains with an enhanced biosynthetic pathway through promoter replacement

Next, to enhance the activity of the biosynthetic genes hisN, hisH, hisD, hisA, and hisB, plasmids were constructed as follows to replace the wild-type promoters of each gene with strong promoters.

Specifically, PCR was performed in the same manner as in Example 1 using *Corynebacterium glutamicum* ATCC13032 chromosomal DNA as a template and primers of SEQ ID NO: 78 and SEQ ID NO: 79, SEQ ID NO: 80 and SEQ ID NO: 81, SEQ ID NO: 82 and SEQ ID NO: 83, SEQ ID NO: 84 and SEQ ID NO: 85 , and SEQ ID NO: 86 and SEQ ID NO: 87, upstream fragments of the hisN, hisH, hisD, hisA, and hisB genes were obtained, respectively.
SEQ ID NO: 78
   - TCGAGCTCGGTACCCATTGGTGCTCGGCGC
SEQ ID NO: 79
   - tgggatgtttctGTGTTGTTAGTCTAGTG
SEQ ID NO: 80
   - TCGAGCTCGGTACCCAACCAAGTTTAGATGCGCC
SEQ ID NO: 81
   - gctgggatgtttctGCCGATAGTTTATGTCA
SEQ ID NO: 82
   - TCGAGCTCGGTACCCGGTGACAGCTCGCGCCGCAT
SEQ ID NO: 83
   - gcgctgggatgtttctGGCGAAAAGTTCTCCC
SEQ ID NO: 84
   - TCGAGCTCGGTACCCTTGATGCCTGCATGAAGG
SEQ ID NO: 85
   - tgacatgaagcgccGAATATTGATCCTATCT
SEQ ID NO: 86
   - TTCGAGCTCGGTACCCACCTTCAGCAACCACTC
SEQ ID NO: 87
   - tgacatgaagcgccGAAAAATTCTTCTCT

In addition, using *Corynebacterium glutamicum* ATCC13032 chromosomal DNA as a template and primer pairs of SEQ ID NO: 88 and SEQ ID NO: 89, SEQ ID NO: 90 and SEQ ID NO: 91, SEQ ID NO: 92 and SEQ ID NO: 93, SEQ ID NO: 94 and SEQ ID NO: 95 , and SEQ ID NO: 96 and SEQ ID NO: 97, downstream regions of the hisN, hisH, hisD, hisA, and hisB genes were obtained, respectively.
SEQ ID NO: 88
   - aaaggaaacactcATGAGCAAATATGCAGACG
SEQ ID NO: 89
   - CTAGAGGATCCCCCAGCCGGAGAGGGAGGAG
SEQ ID NO: 90
   - aaaggaaacactcATGACCAAAACTGTCGC
SEQ ID NO: 91
   - CTAGAGGATCCCCACCTCTGGAGGCGTGGTC
SEQ ID NO: 92
   - cgaaaggaaacactcATGTTGAATGTCACTGACC
SEQ ID NO: 93
   - CTAGAGGATCCCCCCGTGCTCAGCCTGAGGAG
SEQ ID NO: 94
   - ggagatcaaaacaATGACCTTCACTATTCTTCC
SEQ ID NO: 95
   - CTAGAGGATCCCCACGAAACGTGCACAACCTT
SEQ ID NO: 96
   - gagatcaaaacaATGACTGTCGCACCA
SEQ ID NO: 97
   - CTAGAGGATCCCCGGGTCGCGGCCGTAGTGGC

In order to replace the endogenous promoters of the hisN, hisH, and hisD genes with the strong Pcj7 promoter (SEQ ID NO: 14, US 7,662,943 B2), PCR was performed in the same manner as in Example 1 using the genomic DNA of *Corynebacterium stationis* as a template and primers of SEQ ID NO: 98 and SEQ ID NO: 99, SEQ ID NO: 100 and SEQ ID NO: 101, and SEQ ID NO: 102 and SEQ ID NO: 103.
SEQ ID NO: 98
   - ACTAGACTAACAACACagaaacatcccagcgc
SEQ ID NO: 99
   - TCTGCATATTTGCTCATgagtgtttccttt
SEQ ID NO: 100
   - ACATAAACTATCGGCagaaacatcccagcgcta
SEQ ID NO: 101
   - GACAGTTTTGGTCATgagtgtttcctttcg
SEQ ID NO: 102
   - GAGAACTTTTCGCCagaaacatcccagcgct
SEQ ID NO: 103
   - AGTGACATTCAACATgagtgtttcctttcg

In addition, to replace the endogenous promoters of the hisA and hisB genes with the strong Pspl13 promoter (SEQ ID NO: 73, US 10,584,338 B2), PCR was performed in the same manner as in Example 1 using the Pspl13 promoter as a template and primers of SEQ ID NO: 104 and SEQ ID NO: 105, and SEQ ID NO: 106 and SEQ ID NO: 107.
SEQ ID NO: 104
   - AGGATCAATATTCggcgcttcatgtcaac
SEQ ID NO: 105
   - GAATAGTGAAGGTCATtgttttgatctcct
SEQ ID NO: 106
   - GAGAAGAATTTTTCggcgcttcatgtcaa
SEQ ID NO: 107
   - TGGTGCGACAGTCATtgttttgatctcct

After treating the pDC24 vector with restriction enzyme SmaI, recombinant plasmids were each obtained by cloning the amplified upstream DNA fragments of the hisN, hisH, and hisD genes, the Pcj7 promoter fragment, and the downstream DNA fragments of the hisN, hisH, and hisD genes using the Gibson assembly method, and were designated pDC24ΔPn:: Pcj7_hisN, pDC24ΔPn:: Pcj7_hisH, pDC24ΔPn:: Pcj7_hisD, respectively. In addition, after treating the pDC24 vector with the restriction enzyme SmaI, recombinant plasmids were each obtained by cloning the amplified upstream DNA fragments of hisA and hisB genes, the Pspl13 promoter fragment, and the downstream DNA fragments of the hisA and hisB genes using the Gibson assembly method, and were designated pDC24ΔPn:: Pspl13_hisA and pDC24ΔPn:: Pspl13_hisB, respectively. Gibson cloning was performed in the same manner as in Example 1.

The constructed pDC24ΔPn::Pcj7_hisN vector was transformed into the CJ-HIS1 strain constructed in Example 6-1-1 by electroporation. After a second crossover process, a strain was obtained in which the native hisN gene was enhanced by replacing the promoter thereof. The genetic manipulation was confirmed through PCR using primers of SEQ ID NO: 108 and SEQ ID NO: 109 and genome sequencing. The strain thus obtained was designated CJ-HIS2.
SEQ ID NO: 108
   - GAGCATGCATCAAAG
SEQ ID NO: 109
   - AGAAATTTGATCCTTATAA

Sequentially, the constructed pDC24ΔPn::Pcj7_hisH vector was transformed into the CJ-HIS2 strain constructed above by electroporation. After a second crossover process, a strain was obtained in which the native hisH gene was enhanced by replacing the promoter thereof. The genetic manipulation was confirmed through PCR using primers of SEQ ID NO: 110 and SEQ ID NO: 111 and genome sequencing. The strain thus obtained was designated CJ-HIS3.
SEQ ID NO: 110
   - TTGAGAGATGCTTATCG
SEQ ID NO: 111
   - CACTTCAGTGCGGATTCCAA

Sequentially, the constructed pDC24ΔPn::Pcj7_hisD vector was transformed into the CJ-HIS3 strain constructed above by electroporation. After a second crossover process, a strain was obtained in which the native hisD gene was enhanced by replacing the promoter thereof. The genetic manipulation was confirmed through PCR using primers of SEQ ID NO: 112 and SEQ ID NO: 113 and genome sequencing. The strain thus obtained was designated CJ-HIS4.
SEQ ID NO: 112
   - AGCGGGTTTAATTCAGG
SEQ ID NO: 113
   - GTGGGTAAGGGTTTTCGT

Sequentially, the constructed pDC24ΔPn::Pspl13_hisA vector was transformed into the CJ-HIS4 strain constructed above by electroporation. After a second crossover process, a strain was obtained in which the native hisA gene was enhanced by replacing the promoter thereof. The genetic manipulation was confirmed through PCR using primers of SEQ ID NO: 114 and SEQ ID NO: 115 and genome sequencing. The strain thus obtained was designated CJ-HIS5.
SEQ ID NO: 114
   - CACGAAAATGATCGTTTTG
SEQ ID NO: 115
   - TATGGGATTCGATGGCCA

Sequentially, the constructed pDC24ΔPn::Pspl13_hisB vector was transformed into the CJ-HIS5 strain constructed above by electroporation. After a second crossover process, a strain was obtained in which the native hisB gene was enhanced by replacing the promoter thereof. The genetic manipulation was confirmed through PCR using primers of SEQ ID NO: 116 and SEQ ID NO: 117 and genome sequencing. The strain thus obtained was designated CJ-HIS6.
SEQ ID NO: 116
   - TGTGGGAATCGCTGGGCAC
SEQ ID NO: 117
   - CGGTCGCCCGCATCTG

### Example 6-1-3. Production of histidine-producing strains with an enhanced biosynthetic pathway through additional gene insertion

To additionally insert the 'hisE(g1a)G(G233H/T235Q)' gene and the hisD gene, NCgl1021, known as a gene encoding a transposon in *Corynebacterium glutamicum*, was used as the insertion site. Specifically, to construct vectors for NCgl1021 deletion and target gene insertion, PCR was performed in the same manner as in Example 1 using the chromosome of ATCC13032 as a template and primer pairs of SEQ ID NO: 118 and SEQ ID NO: 119, and SEQ ID NO: 120 and SEQ ID NO: 121, thereby amplifying the left homology arm region of NCgl1021 and the right homology arm region of NCgl1021, respectively.
SEQ ID NO: 118
   - TTCGAGCTCGGTACCCATGAAGTCTACCGGC
SEQ ID NO: 119
   - gacatgaagcgccGACATCTAATAACCGGG
SEQ ID NO: 120
   - CCGACGAGGCCTAAGAACTCATTCCTTCTGCT
SEQ ID NO: 121
   - CTCTAGAGGATCCCCTTAGAGTGCATTGATC

PCR was performed in the same manner as in Example 1 using the pDC24ΔPn:: Pspl13_hisEG(G233H/T235Q) vector constructed in Example 6-1-1 as a template and primers of SEQ ID NO: 122 and SEQ ID NO: 123, thereby obtaining the 'Pspl13_hisE(g1a)G(G233H/T235Q)' gene fragment.

In addition, PCR was performed in the same manner as in Example 1 using the pDC24ΔPn:: Pcj7_hisD vector constructed in Example 6-1-2 as a template and primers of SEQ ID NO: 124 and SEQ ID NO: 125, thereby obtaining the 'Pcj7_hisD' gene fragment.
SEQ ID NO: 122
   - CCGGTTATTAGATGTCggcgcttcatgtca
SEQ ID NO: 123
   - ggatgtttctCTAGATGCGGGCGAT
SEQ ID NO: 124
   - GCCCGCATCTAGagaaacatcccagcgct
SEQ ID NO: 125
   - AGAAGGAATGAGTTCTTAGGCCTCGTCGG

After treating the pDC24 vector with restriction enzyme SmaI, a recombinant plasmid was obtained by cloning the amplified left homology arm region of NCgl1021, the right homology arm region of NCgl1021, and the 'Pspl13_hisEG(G233H/T235Q)' and 'Pcj7_hisD' gene fragments using the Gibson assembly method, and was designated 'pDC24ΔNCgl1021:: Pspl13_hisEG(G233H/T235Q)- Pcj7_hisD'. Gibson cloning was performed in the same manner as in Example 1. After transforming the CJ-HIS6 constructed in Example 6-1-2 with the constructed 'pDC24ΔNCgl1021:: Pspl13_hisEG(G233H/T235Q)- Pcj7_hisD' vector by electroporation and undergoing a secondary crossover process, a strain with an enhanced histidine biosynthetic pathway was obtained through the additional insertion of a gene. The genetic manipulation was confirmed through PCR using primers of SEQ ID NO: 126 and SEQ ID NO: 127 and genome sequencing. The strain thus obtained was designated CJH1.
SEQ ID NO: 126
   - CTTTCAGCTTTCCCTCCCG
SEQ ID NO: 127
   - GCTGTACTTTTAGTACA

### Example 6-2. Construction of transformant strains into which the cscK(E.co) gene and glf(Z.mo) gene have been introduced into a histidine-producing strain

In order to determine whether there is an effect of increasing histidine-producing ability through the introduction of a combination of the cscK(E.co) gene and the glf(Z.mo) gene in a *Corynebacterium glutamicum* strain having L-histidine-producing ability, a strain was constructed in which the *ptsF* gene, which imports fructose, was deleted from the histidine-producing strain CJH1 constructed in Example 6-1 above, and the *cscK* gene derived from *Escherichia coli* and the *glf* gene derived from *Zymomonas mobilis* were introduced.

Specifically, PCR was performed in the same manner as in Example 1 using *Corynebacterium glutamicum* ATCC13032 chromosomal DNA as a template and primer pairs of SEQ ID NO: 128 and SEQ ID NO: 129, and SEQ ID NO: 130 and SEQ ID NO: 131. As a result, DNA fragments of 807 bp and 837 bp, respectively, for *ptsF* gene deletion were obtained.
SEQ ID NO: 128
   - ATTCGAGCTCGGTACCCCCTGGCGGGCTCGCTGCC
SEQ ID NO: 129
   - agcgctgggatgtttctTGACCAGGAACGCCGGTGCCGGACT
SEQ ID NO: 130
   - TGGCGCTCCCAGAAGTAGTCTTCGTGGTCTGGGC
SEQ ID NO: 131
   - ACTCTAGAGGATCCCCATTTCTAGGCCCGCA

PCR was performed in the same manner as in Example 1 using the vector 'pDC24ΔBBD29_02180-BBD29_02200:: Pcj7-csck' constructed in Example 2-2 as a template and the primers of SEQ ID NO: 132 and SEQ ID NO: 133.

As a result, a 1270 bp DNA fragment comprising a 1233 bp 'Pcj7-csck' gene fragment was obtained.
SEQ ID NO: 132
   - CCGGCACCGGCGTTCCTGGTCAagaaacatcccagcgcta
SEQ ID NO: 133
   - CGCTGGGATGTTTCTCTATTCCAGTTCTTGTCGACATGGC

PCR was performed in the same manner as in Example 1 using the vector 'pDC24ΔBBD29_12045-BBD29_12055:: Pcj7-glf' constructed in Example 4-3 as a template and the primers of SEQ ID NO: 134 and SEQ ID NO: 135.

As a result, a 1767 bp DNA fragment comprising a 1740 bp 'Pcj7-glf' gene fragment was obtained.
SEQ ID NO: 134
   - CAAGAACTGGAATAGAGAAACATCCCAGCGCT
SEQ ID NO: 135
   - AGACCACGAAGACTACTTCTGGGAGCGCC

Each obtained DNA product was purified using a PCR purification kit, and the purified amplification product and the chromosomal transformation vector pDC24 cleaved with SmaI restriction enzyme were cloned using the Gibson assembly method to obtain a recombinant plasmid, which was designated 'pDC24ΔptsF:: Pcj7-csck-Pcj7-glf'. Gibson cloning was performed in the same manner as in Example 1.

The constructed pDC24ΔptsF:: Pcj7-csck-Pcj7-glf vector was transformed into the histidine-producing CJH1 strain by electroporation. After a secondary crossover process, a strain into which 1 copy of the Pcj7-csck-Pcj7-glf gene was inserted was obtained. The genetic manipulation was confirmed through PCR using primers of SEQ ID NO: 136 and SEQ ID NO: 137 and genome sequencing. The strain thus obtained was designated 'CJH1ΔptsF:: Pcj7-csck-Pcj7-glf'.
SEQ ID NO: 136
   - CAACAAGAACGTCCGCACC
SEQ ID NO: 137
   - TCGAGATCCGTGGGCACTC

### Example 6-3. Evaluation of histidine-producing ability of transformant strains

To confirm the L-histidine-producing ability and fructose utilization ability of the strain constructed in the above example, it was cultured and evaluated using the following method. Each strain was inoculated into a 250-mL corner-baffled flask containing 25 mL of the seed medium below and incubated with shaking at 200 rpm at 30°C for 20 hours. Then, 1 mL of the seed culture was inoculated into a 250-mL corner-baffled flask containing 25 mL of production medium and incubated with shaking at 200 rpm at 30°C for 48 hours.

### [Seed Medium (pH 7.0)]

Glucose 5%, Bactopeptone 1%, Sodium chloride 0.25%, Yeast extract 1%, and Urea 0.4% (based on 1 liter of distilled water)

### [Production Medium (pH 7.0)]

Glucose 3%, Fructose 3%, Ammonium sulfate 2%, Potassium dihydrogen phosphate 0.1%, Magnesium sulfate heptahydrate 0.05%, CSL (Corn steep liquor) 2.0%, Biotin 200 µg/L, and Calcium carbonate 30 g/L (per 1 liter of distilled water)

**[Table 5]**

| Comparison of glucose and fructose utilization and L-histidine-producing ability of L-histidine-producing strains derived from *Corynebacterium glutamicum* ATCC 13032, into which a fructose utilization factor was introduced (48 hr) | | | |
|---|---|---|---|
| Strain No. | Glucose consumed | Fructose consumed | L-histidine |
| | (g/L) | (g/L) | production (g/L) |
| CJH1 | 16.8 | 8.7 | 4.6 |
| CJH1 ΔptsF:: Pcj7-csck-Pcj7-glf | 14.1 | 18.7 | 5.0 |

As shown in the table above, it was confirmed that histidine production increased when the *cscK* gene derived from *Escherichia coli* and the *glf* gene derived from *Zymomonas mobilis* were introduced. Through the above, it was found that the combination of the *cscK* and *glf* genes is effective in increasing L-histidine production.

### Example 7. Evaluation of sugar utilization/L-lysine-producing ability of microorganisms of the genus Corynebacterium, into which the cscK gene derived from a microorganism of the genus Escherichia and the glf gene derived from a microorganism of the genus Zymomonas were introduced

### Example 7-1. Construction of transformant strains into which the cscK(E.co) gene and glf(Z.mo) gene have been introduced into a lysine-producing strain

In order to determine whether there is an effect of increasing lysine-producing ability through the introduction of a combination of the cscK(E.co) gene and the glf(Z.mo) gene in a *Corynebacterium glutamicum* strain having L-lysine-producing ability, a strain was constructed in which the *ptsF* gene, which imports fructose, was deleted from the lysine-producing KCCM11016P strain (US 9,938,546 B2), and the *cscK* gene derived from *Escherichia coli* and the *glf* gene derived from *Zymomonas mobilis* were introduced.

Specifically, the prepared pDC24ΔptsF::Pcj7-csck-Pcj7-glf vector prepared in Example 6-2 was transformed into the lysine-producing KCCM11016P strain by electroporation. After a secondary crossover process, a strain into which 1 copy of the Pcj7-csck-Pcj7-glf gene was inserted was obtained. The corresponding genetic manipulation was confirmed through PCR using primers of SEQ ID NO: 136 and SEQ ID NO: 137, which are capable of amplifying the external regions of the upstream and downstream homologous recombination sites where the corresponding gene has been inserted, respectively, and through genome sequencing. The strain thus obtained was designated 'KCCM11016PΔptsF:: Pcj7-csck-Pcj7-glf'.

### Example 7-2. Evaluation of lysine-producing ability of transformant strains

To confirm the L-lysine-producing ability and fructose utilization ability of the strain constructed in the above example, it was cultured and evaluated using the following method. Each strain was inoculated into a 250-mL corner-baffled flask containing 25 mL of the seed medium below and incubated with shaking at 200 rpm at 37°C for 20 hours. Then, 1 mL of the seed culture was inoculated into a 250-mL corner-baffled flask containing 24 mL of production medium and incubated with shaking at 200 rpm at 37°C for 42 hours.

### [Seed Medium (pH 7.0)]

Raw sugar 20 g, Peptone 10 g, Yeast extract 5 g, Urea 1.5 g, KH₂PO₄ 4 g, K₂HPO₄ 8 g, MgSO₄·7H₂O 0.5 g, Biotin 0.1 mg, Thiamine HCl 1 mg, Calcium pantothenate 22 mg, and Nicotinamide 2 mg (based on 1 liter of distilled water)

### [Production Medium (pH 7.0)]

Glucose 22.5 g, Fructose 22.5 g, (NH4)2SO4 30 g, Soy protein 10 g, 50% Sugar molasses 10 g, KH₂PO₄ 0.55 g, MgSO₄·7H₂O 0.6 g, Biotin 0.9 mg, Thiamine hydrochloride 4.5 mg, Calcium pantothenic acid 4.5 mg, Nicotinamide 30 mg, MnSO4 9 mg, FeSO4 9 mg, ZnSO4 0.45 mg, CuSO4 0.45 mg, and CaCO3 30 g (based on 1 liter of distilled water)

**[Table 6]**

| Comparison of glucose and fructose utilization and L-lysine-producing ability of L-lysine producing strains derived from *Corynebacterium glutamicum* ATCC13032, into which a fructose utilization factor was introduced (42 hr) | | | |
|---|---|---|---|
| Strain No. | Glucose consumed (g/L) | Fructose consumed (g/L) | L-lysine production (g/L) |
| KCCM11016P | 22.5 | 22.5 | 11.9 |
| KCCM11016P ΔptsF:: Pcj7-csck-Pcj7-glf | 22.5 | 22.5 | 13.0 |

As shown in the table above, it was confirmed that lysine production increased when the *cscK* gene derived from *Escherichia coli* and the *glf* gene derived from *Zymomonas mobilis* were introduced. Through the above, it was found that the combination of the *cscK* and *glf* genes is effective in increasing L-lysine production.

### Example 8. Evaluation of sugar utilization/L-threonine-producing ability of Corynebacterium microorganisms into which the cscK gene derived from Escherichia genus microorganisms and the glf gene derived from Zymomonas genus microorganisms were introduced

### Example 8-1. Construction of transformant strains into which the cscK(E.co) gene and glf(Z.mo) gene have been introduced into a threonine-producing strain

In order to determine whether there is an effect of increasing threonine-producing ability through the introduction of a combination of the cscK(E.co) gene and the glf(Z.mo) gene in a *Corynebacterium glutamicum* strain having L-threonine-producing ability, a strain was constructed in which the *ptsF* gene, which imports fructose, was deleted from the threonine-producing KCCM12120P strain (US 11,236,374 B2), and the *cscK* gene derived from *Escherichia coli* and the *glf* gene derived from *Zymomonas mobilis* were introduced.

Specifically, the prepared pDC24ΔptsF:: Pcj7-csck-Pcj7-glf vector prepared in Example 6-2 was transformed into the threonine-producing KCCM12120P strain by electroporation. After a secondary crossover process, a strain into which 1 copy of the Pcj7-csck-Pcj7-glf gene was inserted was obtained. The corresponding genetic manipulation was confirmed through PCR using primers of SEQ ID NO: 136 and SEQ ID NO: 137, which are capable of amplifying the external regions of the upstream and downstream homologous recombination sites where the corresponding gene has been inserted, respectively, and through genome sequencing. The strain thus obtained was designated 'KCCM12120PΔptsF:: Pcj7-csck-Pcj7-glf'.

### Example 8-2. Evaluation of threonine-producing ability of transformant strains

To confirm the L-threonine-producing ability and fructose utilization ability of the strain constructed in the above example, it was cultured and evaluated using the following method. Each strain was inoculated into a 250-mL corner-baffled flask containing 25 mL of the seed medium and incubated with shaking at 200 rpm at 30°C for 20 hours. Then, 1 mL of the seed culture was inoculated into a 250-mL corner-baffled flask containing 24 mL of production medium and incubated with shaking at 200 rpm at 30°C for 20 hours.

### [Seed Medium (pH 7.0)]

Glucose 20 g, Peptone 10 g, Yeast extract 5 g, Urea 1.5 g, KH₂PO₄ 4 g, K₂HPO₄ 8 g, MgSO₄·7H₂O 0.5 g, Biotin 100 µg, Thiamine HCl 1000 µg, Calcium pantothenate 2000 µg, and Nicotinamide 2000 µg (per 1 L of distilled water).

### [Production Medium (pH 7.0)]

Glucose 15 g, Fructose 15 g, KH₂PO₄ 2 g, Urea 3 g, (NH₄)₂SO₄ 40 g, Peptone 2.5 g, CSL(Sigma) 5 g (10 mL), MgSO₄·7H₂O 0.5 g, Leucine 400 mg, and CaCO3 20 g (based on 1 liter of distilled water)

**[Table 7]**

| Comparison of glucose and fructose utilization and L-threonine-producing ability of L-threonine producing strains derived from *Corynebacterium glutamicum* ATCC13032, into which a fructose utilization factor was introduced (48 hr) | | | |
|---|---|---|---|
| Strain No. | Glucose consumed (g/L) | Fructose consumed (g/L) | L-Threonine production (g/L) |
| KCCM 12120P | 15 | 15 | 1.01 |
| KCCM 12120P Δ ptsF:: Pcj7-csck-Pcj7-glf | 15 | 15 | 1.3 |

As shown in the table above, it was confirmed that threonine production increased when the *cscK* gene derived from *Escherichia coli* and the *glf* gene derived from *Zymomonas mobilis* were introduced. Through the above, it was found that the combination of the *cscK* and *glf* genes is effective in increasing L-threonine production.

### Example 9. Evaluation of L-amino acid-producing ability of microorganisms of the genus Corynebacterium, into which a variant cscK gene derived from a microorganism of the genus Escherichia and the glf gene derived from a microorganism of the genus Zymomonas were introduced

### Example 9-1. Vector Construction

To determine whether the introduction of a combination of a variant *cscK* gene (cscK_W79C or cscK_W79C/A181V) and the glf(Z.mo) gene into a *Corynebacterium glutamicum* strain having L-amino acid-producing ability has an effect of increasing amino acid-producing ability, a vector was constructed to introduce the *cscK* gene variants derived from *Escherichia coli*.

Specifically, PCR was performed in the same manner as in Example 1 using the vector 'pDC24ΔBBD29_02180-BBD29_02200:: Pcj7-csck' constructed in Example 2-2 as a template and the primer pair of SEQ ID NO: 141 and SEQ ID NO: 142, and the primer pair of SEQ ID NO: 143 and SEQ ID NO: 144. Overlapping PCR was performed again using the mixture of the two fragments obtained as a template and the primer pair of SEQ ID NO: 141 and SEQ ID NO: 144, and as a result, a DNA fragment for introducing the variant csck(W79C, E.co) (SEQ ID NO: 147) was obtained.

In addition, PCR was performed in the same manner as in Example 1 using the vector 'pDC24ΔBBD29_02180-BBD29_02200:: Pcj7-csck' constructed in Example 2-2 as a template and the primer pair of SEQ ID NO: 141 and SEQ ID NO: 142, the primer pair of SEQ ID NO: 143 and SEQ ID NO: 145, and the primer pair of SEQ ID NO: 146 and SEQ ID NO: 144. Overlapping PCR was performed again using the mixture of the three fragments obtained as a template and the primer pair of SEQ ID NO: 141 and SEQ ID NO: 144, and as a result, a DNA fragment for introducing the variant cscK(W79C/A181V, E.co) (SEQ ID NO: 148) was obtained.
SEQ ID NO: 141
   - TCGAGCTCGGTACCCccaccacctaaaaat
SEQ ID NO: 142
   - CGTGGATGTCCGGTGACATTCATCTTGCTT
SEQ ID NO: 143
   - CTGAAGCAAGATGAATGTCACCGGACATCC
SEQ ID NO: 144
   - CTCTAGAGGATCCCCGATAATTCGTCGCATTTC
SEQ ID NO: 145
   - AGAGCTTGACGACATCCACCAGTTGTAGCGC
SEQ ID NO: 146
   - GCGCTACAACTGGTGGATGTCGTCAAGCTCT

The obtained DNA products were purified using a PCR purification kit, and the purified amplification products and the chromosomal transformation vector pDC24 cleaved with SmaI restriction enzyme were cloned using the Gibson assembly method to obtain recombinant plasmids, which were designated 'pDC24Δcsck(E.co):: csck(W79C, E.co)' and 'pDC24Δcsck(E.co):: csck(W79C/A181V, E.co)', respectively. Gibson cloning was performed in the same manner as in Example 1.

### Example 9-2. Strain construction

After transforming each of the recombinant vectors 'pDC24Δcsck(E.co)::csck(W79C,E.co)' and 'pDC24Δcsck(E.co)::csck(W79C/A181V, E.co)' constructed in Example 9-1 into the production strains CM05-9841, 'CJH1ΔptsF::Pcj7-csck-Pcj7-glf', 'KCCM11016PΔptsF::Pcj7-csck-Pcj7-glf', and 'KCCM12120PΔptsF::Pcj7-csck-Pcj7-glf' constructed in Examples 4-3, 6-2, 7-1, and 8-1 by the electro-pulse method. After a second crossover process, strains in which the wild-type csck(E.co) gene on the chromosome was replaced with the variant csck(W79C, E.co) or csck(W79C/A181V, E.co) gene were obtained. These strains were designated CM05-9841Δcsck(E.co)::csck(W79C, E.co), CJH1ΔptsF::Pcj7-csck(W79C)-Pcj7-glf, KCCM11016PΔptsF::Pcj7-csck(W79C)-Pcj7-glf, KCCM12120PΔptsF::Pcj7-csck(W79C)-Pcj7-glf, CM05-9841Δcsck(E.co)::csck(W79C/A181V, E.co), CJH1ΔptsF::Pcj7-csck(W79C/A181V)-Pcj7-glf, KCCM11016PΔptsF::Pcj7-csck(W79C/A181V)-Pcj7-glf, and KCCM12120PΔptsF::Pcj7-csck(W79C/A181V)-Pcj7-glf, respectively.

### Example 9-3. Producing-ability evaluation

To confirm the L-amino acid-producing ability of the strains produced in the above examples, they were cultured and evaluated in the same manner as in Examples 5, 6-3, 7-2, and 8-2.

**[Table 8]**

| Comparison of the L-tryptophan-producing ability of L-tryptophan-producing strains (20 hr) | |
|---|---|
| Strain No. | L-tryptophan production (g/L) |
| CM05-9841 | 2.2 |
| CM05-9841Δcsck(E.co)::csck(W79C,E.co) | 2.8 |
| CM05-9841Δcsck(E.co)::csck(W79C/A181V,E.co) | 2.9 |

**[Table 9]**

| Comparison of the L-histidine-producing ability of L-histidine-producing strains (48 hr) | |
|---|---|
| Strain No. | L-histidine production (g/L) |
| CJH1 ΔptsF::Pcj7-csck-Pcj7-glf | 5.0 |
| CJH1 ΔptsF::Pcj7-csck(W79C)-Pcj7-glf | 5.5 |
| CJH1 ΔptsF::Pcj7-csck(W79C/A181V)-Pcj7-glf | 5.6 |

**[Table 10]**

| Comparison of the L-lysine-producing ability of L-lysine-producing strains (42 hr) | |
|---|---|
| Strain No. | L-lysine production (g/L) |
| KCCM 11016P ΔptsF::Pcj7-csck-Pcj7-glf | 13.0 |
| KCCM 11016P ΔptsF::Pcj7-csck(W79C)-Pcj7-glf | 14.0 |
| KCCM 11016P ΔptsF::Pcj7-csck(W79C/A181V)-Pcj7-glf | 13.8 |

**[Table 11]**

| Comparison of L-threonine-producing ability of L-threonine producing strains (48 hr) | |
|---|---|
| Strain No. | L-Threonine production (g/L) |
| KCCM 12120P ΔptsF::Pcj7-csck-Pcj7-glf | 1.3 |
| KCCM 12120P ΔptsF::Pcj7-csck(W79C)-Pcj7-glf | 1.8 |
| KCCM 12120P ΔptsF::Pcj7-csck(W79C/A181V)-Pcj7-glf | 1.6 |

As shown in the tables above, it was confirmed that the production of amino acids increased when a variant *cscK* gene derived from *Escherichia coli* was introduced. Through the above, it was found that the combination of a variant *cscK* and the *glf* gene is effective in increasing L-amino acid production.

As set forth above, those skilled in the art will be able to understand that the present disclosure may be embodied in other specific forms without departing from the technical spirit or essential characteristics thereof. In this regard, it should be understood that the foregoing examples are illustrative in all respects and are not to be construed as limiting. The scope of the present disclosure should be construed as comprising the meaning and scope of the appended claims rather than the detailed description, and all changes or variations derived from the equivalent concepts fall within the scope of the present disclosure.

## Claims

1. A microorganism of the genus *Corynebacterium* that produces an L-amino acid, comprising: at least one selected from the group consisting of a fructokinase derived from a microorganism of the genus *Escherichia*, a polynucleotide encoding the same, a variant polypeptide of the fructokinase, and a polynucleotide encoding the variant polypeptide; and at least one selected from the group consisting of a non-PTS sugar transporter derived from a microorganism of the genus *Zymomonas*, and a polynucleotide encoding the same.

2. The microorganism of the genus *Corynebacterium* according to claim 1, wherein the microorganism has increased L-amino acid-producing ability compared to a nonmodified microorganism.

3. The microorganism of the genus *Corynebacterium* according to claim 1, wherein the fructokinase is encoded by *cscK* gene.

4. The microorganism of the genus *Corynebacterium* according to claim 1, wherein the fructokinase is encoded by *cscK* gene derived from *Escherichia coli*.

5. The microorganism of the genus *Corynebacterium* according to claim 1, wherein the fructokinase comprises an amino acid sequence of SEQ ID NO: 139 or an amino acid sequence having at least 80% sequence identity thereto.

6. The microorganism of the genus *Corynebacterium* according to claim 1, wherein the variant polypeptide has an amino acid substitution at the position corresponding to position 79 or position 181 of SEQ ID NO: 139 with a different amino acid.

7. The microorganism of the genus *Corynebacterium* according to claim 6, wherein the variant polypeptide has an amino acid substitution at the position corresponding to position 79 of SEQ ID NO: 139 with cysteine, or an amino acid substitution at the position corresponding to position 181 of SEQ ID NO: 139 with valine, or a combination thereof.

8. The microorganism of the genus *Corynebacterium* according to claim 6, wherein the variant polypeptide comprises an amino acid sequence of SEQ ID NO: 147 or an amino acid sequence having at least 80% sequence identity thereto; or an amino acid sequence of SEQ ID NO: 148 or an amino acid sequence having at least 80% sequence identity thereto.

9. The microorganism of the genus *Corynebacterium* according to claim 1, wherein the non-PTS sugar transporter has glucose and fructose uptake ability.

10. The microorganism of the genus *Corynebacterium* according to claim 1, wherein the non-PTS sugar transporter is encoded by *glf* gene.

11. The microorganism of the genus *Corynebacterium* according to claim 1, wherein the non-PTS sugar transporter is encoded by *glf* gene derived from *Zymomonas mobilis*.

12. The microorganism of the genus *Corynebacterium* according to claim 1, wherein the non-PTS sugar transporter comprises an amino acid sequence of SEQ ID NO: 140 or an amino acid sequence having at least 80% sequence identity thereto.

13. The microorganism according to claim 1, wherein the microorganism of the genus *Corynebacterium* is *Corynebacterium glutamicum*.

14. The microorganism of the genus *Corynebacterium* according to claim 1, wherein the microorganism of the genus *Corynebacterium* has a deletion of *ptsF* gene.

15. The microorganism of the genus *Corynebacterium* according to claim 1, wherein the L-amino acid is at least one selected from the group consisting of L-tryptophan, L-lysine, L-histidine, and L-threonine.

16. A composition for producing an L-amino acid, comprising the microorganism according to claim 1, a culture of the microorganism, a fermentation product of the microorganism, or a combination of two or more thereof.

17. A method for producing an L-amino acid, comprising culturing, in a medium, a microorganism of the genus *Corynebacterium* comprising: at least one selected from the group consisting of a fructokinase derived from a microorganism of the genus *Escherichia*, a polynucleotide encoding the same, a variant polypeptide of the fructokinase, and a polynucleotide encoding the variant polypeptide; and at least one selected from the group consisting of a non-PTS sugar transporter derived from a microorganism of the genus *Zymomonas*, and a polynucleotide encoding the same.

18. The method for producing an L-amino acid according to claim 17, further comprising recovering the L-amino acid from the cultured microorganism, the culture of the microorganism, the fermentation product of the microorganism, or the culture medium.

19. Use of a microorganism of the genus *Corynebacterium* for producing an L-amino acid, wherein the microorganism comprises: at least one selected from the group consisting of a fructokinase derived from a microorganism of the genus *Escherichia*, a polynucleotide encoding the same, a variant polypeptide of the fructokinase, and a polynucleotide encoding the variant polypeptide; and at least one selected from the group consisting of a non-PTS sugar transporter derived from a microorganism of the genus *Zymomonas*, and a polynucleotide encoding the same.
